# EUROPÄISCHE PATENTSCHRIFT

(11) **EP 4 702 947 B1**
(45) Veröffentlichungstag und Bekanntmachung des Hinweises auf die Patenterteilung: **28.10.2026**
(21) Anmeldenummer: 24197511.9
(22) Anmeldetag: 30.08.2024
(51) Int. Cl.: A61C 5/77, A61C 13/00, A61C 13/083, C04B 35/00

(54) **VERFAHREN ZUR HERSTELLUNG KERAMISCHER UND GLASKERAMISCHER ZAHNRESTAURATIONEN**
METHOD FOR PRODUCING CERAMIC AND GLASS-CERAMIC DENTAL RESTORATIONS
PROCÉDÉ DE FABRICATION DE RESTAURATIONS DENTAIRES CÉRAMIQUES ET VITROCÉRAMIQUES

(43) Veröffentlichungstag der Anmeldung: 04.03.2026
(73) Patentinhaber: Ivoclar Vivadent AG, 9494 Schaan (LI)
(72) Erfinder: Cavegn, Nadja, 9498 Planken (LI); Bonderer, Lorenz Josef, 7320 Sargans (CH)
(74) Vertreter: Uexküll & Stolberg

(56) Entgegenhaltungen:
- US-A1- 2011 212 419
- US-B2- 8 813 364
- US-B2- 8 973 268

## Beschreibung

Die vorliegende Erfindung betrifft ein Verfahren zur Herstellung keramischer und glaskeramischer Zahnrestaurationen, wie dentalen Inlays, Onlays, Veneers, Kronen, Brücken und Gerüsten.

Aus ästhetischen Gründen werden Dentalrestaurationen in zunehmendem Maße vollständig aus Keramik hergestellt. In der Praxis wird hierzu häufig ein Gerüst aus Oxidkeramik mit Glaskeramik verblendet.

Die DE 10 2005 042 091 A1 offenbart ein Verfahren zur Herstellung von keramischem Zahnersatz, bei dem ein Gerüst aus einem oxidkeramischen Rohling, beispielsweise aus Aluminiumoxid oder Zirkoniumdioxid, und eine Verblendung aus einem Glaskeramikblock, beispielsweise aus Silikatkeramik, gefräst werden. Die Herstellung von Gerüst und Verblendung erfolgen mittels CAD/CAM-Verfahren. Gerüst und Verblendung werden anschließend unter Verwendung einer dünnflüssigen, niedrig schmelzenden Keramikmasse zusammengefügt und gebrannt. Nach dem Zusammensintern wird die Okklusion eingeschliffen und die Restauration in einem Glanzbrand fertiggestellt.

Die WO 2007/051447 A1 offenbart ein Verfahren zur Herstellung von mehrschichtig aufgebautem Zahnersatz, bei dem mittels CAD/CAM zunächst eine tragende Gerüststruktur aus metallischen oder keramisch Werkstoffen oder Polymeren herstellt und dann eine zur Verblendung dienende Funktionsschicht aufgetragen wird. Die Funktionsschicht wird vorzugsweise durch additive Verfahren wie Heißpressen, Druckguss oder Schlickerguss hergestellt.

Die Herstellung von keramischen Formteilen durch Fräsen ist nachteilig, weil Keramikmaterialien sehr fest sind und die Bearbeitung damit sehr zeitaufwendig und mit einem hohen Verschleiß der verwendeten Schleifgeräte und Fräsen verbunden ist. Die WO 2010/010087 A1 schlägt zur Vermeidung dieser Nachteile die Verwendung poröser Keramikkörper zur Herstellung von Verblendschalen vor, die auf ein Gerüst aus Metall oder Keramik aufgebracht werden. Die Verblendschalen können aus mehreren Schichten bestehen, deren Grenzfläche dem Verlauf der Dentin-/Schmelzgrenze natürlicher oder künstlicher Zähne entspricht. Sie werden hergestellt, indem handelsübliche Verblendkeramikpulver durch Pressen zu Blöcken geformt und dann vorgesintert werden. Aus den Blöcken werden mit einem CAD/CAM-System Verblendschalen gefräst, die dann auf Zirkondioxidkäppchen aufgebrannt und dabei dichtgesintert werden. Bei dieser Vorgehensweise ist das unterschiedliche Schrumpfungsverhalten von Gerüst- und Verblendmaterial zu berücksichtigen.

Die DE 10 2010 002 484 A1 offenbart die Herstellung von Zahnkronen mit einem Kern aus Oxidkeramik und einer Außenschicht aus Kunststoff, Oxid- oder Glaskeramik. Die Grenzfläche zwischen Kern und Außenschicht folgt dem Verlauf der Dentin-Schmelz-Grenze eines natürlichen Zahns. Auf diese Weise soll ein dem natürlichen Zahn entsprechendes Erscheinungsbild erreicht werden. Die Herstellung des Kerns erfolgt mittels computergestützter Verfahren, beispielsweise CAD/CAM oder generative Verfahren, die Herstellung der Außenschicht durch manuelles Aufschichten, Überpressen oder mittels computergestützter Verfahren. Die Außenschicht wird durch Aufkleben, Aufsintern oder Aufpolymerisieren mit dem Kern verbunden.

Die DE 10 2016 115 916 A1 offenbart ein Verfahren zur Erzeugung von Bearbeitungsdaten zur Herstellung von Zahnprothesen mit einer keramischen Verblendschale und einem Träger. Das Verfahren berücksichtigt unterschiedliche Schrumpfungsfaktoren für den Träger und die Verblendschale und soll erstmals das gemeinsame Sintern von Verblendschalen und Träger aus unterschiedlichen Materialien ermöglichen. Problematisch ist, dass sich Träger und Verblendschale erst beim Endsintern vollständig aneinander anpassen, was ein genaues Positionieren der Schale auf dem Träger vor dem Sintern erschwert und das Fehlerrisiko erhöht.

Neben klassischen Fräsverfahren werden zunehmend generative Verfahren, wie z.B. die Stereolithographie, zur Herstellung von Dentalrestaurationen eingesetzt. Bei der Stereolithographie wird ein Formteil unter Verwendung computergestützter Konstruktionsdaten (CAD-Daten) schichtweise aus einem flüssigen und härtbaren Monomerharz aufgebaut (A. Beil, Fertigung von Mikro-Bauteilen mittels Stereolithographie, Düsseldorf 2002, VDI-Verlag 3 ff.). Bei der Herstellung von dentalen Formkörpern aus keramischen Werkstoffen sind stereolithographische Verfahren vorteilhaft, weil sie eine deutliche Vereinfachung der manuellen Abform- und Gießprozesse sowie der Fräs- und Schleifoperationen ermöglichen und den bei nicht-generativen Verfahren auftretenden große Materialverlust vermeiden. Da heutzutage eine vollständige digitale Prozesskette etabliert ist, lassen sich die klassischen Verfahrensschritte zur Herstellung von Dentalrestaurationen durch die Digitalisierung des Modells, die virtuelle Konstruktion des dentalen Formkörpers und dessen generative stereolithographische Fertigung ersetzen.

Zur stereolithographischen Herstellung von keramischen Formteilen wird zunächst ein keramischer Grünling durch schichtweise Strahlungshärtung eines keramischen Schlickers hergestellt, der dann nach der Entbinderung zu einem dichten keramischen Formkörper gesintert wird. Der Grünling wird auch Grünkörper genannt. Als Entbinderung wird das Austreiben des Bindemittels bezeichnet. Hierbei wird das verwendete Bindemittel meist durch Erhitzen des Grünlings auf eine Temperatur von ca. 80°C bis 600°C durch thermische und thermo-chemische Prozesse zu flüchtigen Komponenten abgebaut und entfernt. Wesentlich ist, dass die Bildung von Rissen und Deformationen weitestgehend vermieden wird.

Das Entbindern ist ein kritischer Schritt des Verfahrens. Hier ist die Gefahr groß, dass das Bauteil durch Gase, die bei der Zersetzung der organischen Matrix entstehen, und den durch diese ausgeübten Druck beschädigt wird. Schon kleine Defekte zwischen den einzelnen Bauschichten können beim Entbindern zu Rissen oder gar zur vollständigen Zerstörung des Bauteils führen. Dieses Risiko kann dadurch reduziert werden, dass die Entbinderungsdauer erhöht wird, was jedoch die Prozesszeit stark verlängert.

Die Sinterung des entbinderten Bauteils erfolgt beim Hochtemperaturbrand im Sinterofen. Dabei kommt es zur Verdichtung und Verfestigung des fein verteilten Keramikpulvers durch Temperatureinwirkung unterhalb der Schmelztemperatur der Hauptkomponenten, wodurch das poröse Bauteil kleiner wird und dessen Festigkeit zunimmt.

Die US 5,496,682 offenbart lichthärtbare Zusammensetzungen zur Herstellung dreidimensionaler Körper durch Stereolithographie, die 40 bis 70 Vol.-% Keramik- oder Metallpartikel, 10 bis 35 Gew.-% Monomer, 1 bis 10 Gew.-% Photoinitiator, 1 bis 10 Gew.-% Dispergiermittel und vorzugsweise auch Lösungsmittel, Weichmacher und Kopplungsmittel enthalten.

Die US 6,117,612 beschreibt Harze zur stereolithographische Herstellung von gesinterten Keramik- oder Metallteilen. Die Harze haben eine Viskosität von weniger als 3000 mPa·s. Zu ihrer Herstellung werden Monomere mit geringer Viskosität eingesetzt, vorzugsweise in wässriger Lösung. Durch die Verwendung von Dispersionsmitteln soll ein hoher Feststoffgehalt bei geringer Viskosität erzielt werden.

Die DE 10 2005 058 116 A1 offenbart Suspensionen zur stereolithographischen Herstellung von keramischen Implantaten auf die in der US 6,117,612 beschriebene Weise, die keine Verdünnungsmittel wie Wasser oder organische Lösungsmittel enthalten. Die Viskosität der Suspension wird durch Variation der Konzentration eines Dispergators auf weniger als 20 Pa·s eingestellt. Als Dispergator werden Alkylammoniumsalze von Copolymeren mit sauren Gruppen eingesetzt, wobei diese auch auf die Partikel des keramischen Pulvers geschichtet werden können.

In der US 2005/0090575 A1 werden Methoden und Zusammensetzungen für die stereolithographische Herstellung von keramischen Bauteilen beschrieben. Es wird angegeben, dass mit den aus US 5,496,682 bekannten flüssigen Materialien hergestellte Formteile weich sind und daher einen zusätzlichen Härtungsschritt erfordern, um Verformungen beim Brennen zu vermeiden, während aus pastenförmigen Materialien gewonnene Formkörper bei der Entbinderung innere Spannungen aufbauen, die beim Sintern zu Rissen führen. Zur Vermeidung dieser Probleme werden Weichmacher eingesetzt und die Menge des Keramikpulvers so gewählt, dass die Viskosität der Zusammensetzungen mindestens 10.000 Pa·s beträgt.

Aus der EP 2 233 449 A1 sind Schlicker zur Herstellung von Keramikformteilen durch Hot-Melt-Inkjet-Druckverfahren bekannt, die Keramikpartikel, Wachs und mindestens ein radikalisch polymerisierbares Wachs enthalten und die Grünkörper ergeben, die sich ohne Rissbildung entbindern lassen. Nachteilig bei diesen Schlickern ist, dass sie im flüssigen Zustand bei längerem Stehen zur Entmischung neigen. Dies ist bei Hot-Melt-Inkjet-Verfahren unkritisch, weil die Schlicker nur während des Druckvorgangs, d.h. für einen relativ kurzen Zeitraum in flüssiger Form vorliegen. Bei stereolithographischen Verfahren müssen die Schlicker jedoch über längere Zeiträume in flüssiger Form stabil sein, d.h. insbesondere, dass sich die im Schlicker dispergierten Partikel nicht vorzeitig absetzen dürfen.

US2011212419 offenbart ein Verfahren, eine Vorrichtung und ein Computerprogramm zur Herstellung einer Dentalprothese.

Der Erfindung liegt die Aufgabe zugrunde, Verfahren und Materialien zur Herstellung vollkeramischer Dentalrestaurationen mit hoher Festigkeit bereitzustellen, die die Nachteile des Standes der Technik nicht aufweisen und die eine vereinfachte Herstellung ästhetisch anspruchsvoller Restaurationen mit natürlicher Optik ermöglichen.

Erfindungsgemäß wird diese Aufgabe durch ein Verfahren gelöst, bei dem man Dentalrestaurationen mit einer einteiligen Gerüststruktur und einer dazu passenden Verblendstruktur herstellt, indem man die Gerüststruktur und die Verblendstruktur getrennt voneinander mit einem additiven Verfahren herstellt und anschließend zu der Dentalrestauration zusammengefügt. Die Gerüststruktur wird hier auch als Corestruktur oder Core bezeichnet. Die Verblendstruktur kann einteilig sein oder mehrere Komponenten umfassen, die zusammen die Verblendstruktur bilden. Die Verblendstruktur und deren Komponenten werden auch als Shellstruktur oder Shell bezeichnet. Wenn die Verblendstruktur mehrere Komponenten umfasst, werden diese vorzugsweise ebenfalls getrennt voneinander hergestellt.

Im ersten Schritt des erfindungsgemäßen Verfahrens wird mit einem CAD (Computer-Aided Design)-Programm ein digitales Konstruktionsmodell der dentalen Restauration gemäß den anatomischen und klinischen Anforderungen konstruiert. Anschließend unterteilt man den hierbei erhaltenen CAD-Datensatz der Restauration in mindestens zwei separate, aber nicht voneinander unabhängige Teildatensätze (Filesplitting), wobei ein erster CAD-Teildatensatz die Konturen der inneren Kernstruktur (Core) und ein zweiter CAD-Teildatensatz die Konturen der äußeren Schalenstruktur (Shell) der Dentalrestauration definiert. Der zweite Teildatensatz kann seinerseits in zwei oder mehr Teildatensätze unterteilt werden, die jeweils einen Teil der Kontur der äußeren Schalenstruktur umfassen. Zwischen den Konturen können an definierten Stellen ein oder mehrere Fügespalte vorgesehen sein. Als Ergebnis werden mindestens zwei CAD-Datensätze erhalten, die durch die CAD-Software jeweils in einem geeigneten Dateiformat, vorzugsweise im STL-Format, abgespeichert werden. Auf Basis der CAD-Datensätze werden anschließend mit einer CAM (Computer-Aided Manufacturing)-Software Bearbeitungsdaten zur additiven Fertigung der Core- und Shellstruktur(en) erzeugt. Das finale Ausgabeformat sind mindestens zwei separate Ausgabedateien. Core- und Shellstruktur(en) werden im anschließenden Folgeprozess in einem computergesteuerten Verfahren mittels der erstellten Datensätze unabhängig voneinander additiv gefertigt, vorzugsweise durch Stereolithographie.

Core- und Shellstruktur(en) werden den gewünschten Anforderungen entsprechend konstruiert. Die Corestruktur ist gewöhnlich intensiver gefärbt und weniger transluzent als die weniger farbige aber transluzentere Shellstruktur. Deren jeweilige Ausprägungen werden individuell den ästhetischen Ansprüchen des Behandlers oder des Patienten bzw. den Gegebenheiten des Restzahnbestandes angepasst.

Zur Fertigung der Dentalrestauration stellt man vorzugsweise mittels des ersten CAD-Teildatensatzes einen Grünling der Corestruktur der Restauration her, indem man einen ersten Schlicker durch lokales Einbringen von Strahlungsenergie unter Ausbildung der geometrischen Form der Corestruktur aushärtet.

In einem weiteren Schritt stellt man mittels des zweiten CAD-Teildatensatzes einen Grünling einer Shellstruktur der Restauration her, indem man einen zweiten Schlicker durch lokales Einbringen von Strahlungsenergie unter Ausbildung der geometrischen Form der Shellstruktur aushärtet.

Wenn die Shellstruktur mehr als eine Komponente umfasst, stellt man separat jeweils einen Grünling für jeden Teil der Shellstruktur her. Zur Herstellung mehrteiliger Shellstrukturen unterteilt man den zweiten CAD-Teildatensatz des digitalen Konstruktionsmodells in zwei oder mehr weitere CAD-Datensätze (n_{Ausgabedatei} > 2), die jeweils einen Teil der Kontur der Shellstruktur umfassen. Die Herstellung der Komponenten erfolgt dann unter Verwendung dieser CAD-Shellstrukturteildatensätze, indem man einen zweiten Schlicker mittels des ersten CAD-Shellstrukturteildatensatzes durch lokales Einbringen von Strahlungsenergie unter Ausbildung der geometrischen Form einer ersten Komponente der Shellstruktur aushärtet, man getrennt davon einen weiteren Schlicker mittels eines zweiten CAD-Shellstrukturteildatensatzes durch lokales Einbringen von Strahlungsenergie unter Ausbildung der geometrischen Form einer zweiten Komponente der Shellstruktur aushärtet, und man diesen Schritt der Anzahl der CAD-Shellstrukturteildatensätze entsprechend so oft wie nötig wiederholt, um die gewünschte Anzahl an Komponenten herzustellen. Pro Wiederholung erhält man einen Grünling einer Komponente der Shellstruktur.

Zur Herstellung der Komponenten der Shellstruktur kann man in allen Fällen denselben oder für jede Komponente einen anderen Schlicker verwenden. Vorzugsweise werden die Komponenten aus unterschiedlichen, besonders bevorzugt aus unterschiedlich gefärbten Schlickern hergestellt.

In einem folgenden Schritt fügt man Core- und Shellstruktur oder Corestruktur und die Komponenten der Shellstruktur im Grünzustand zusammen, um einen zusammengefügten Grünling der Dentalrestauration zu erhalten. Vorzugsweise wird hierbei ein weiterer Schlicker zur Verbesserung des Verbunds der Einzelteile eingesetzt, der hier als Fügeschlicker bezeichnet wird. Der Fügeschlicker ist vorzugsweise polymerisierbar, besonders bevorzugt radikalisch polymerisierbar. Ganz besonders bevorzugt sind photoreaktive Fügeschlicker, d.h. Schlicker, die sich durch die Bestrahlung mit Licht härten lassen, und die Polymerisation des Fügeschlickers wird vorzugsweise durch die Belichtung des Schlickers initiiert. Die Polymerisation des Fügeschlickers ermöglicht einen festen Verbund der gefügten Einzelelemente der dentalen Restauration im Grünzustand.

Vor dem Zusammenfügen der Teile und bevor der Fügeschlicker applizitiert wird, können die Fügeflächen der Core- und/oder Shellstruktur durch das gezielte Aufbringen intensiv eingefärbter Schlicker individuell charakterisiert werden. Hierzu eignen sich insbesondere Schlicker, deren Zusammensetzung mit den zur Herstellung von Core- und Shellstruktur verwendeten Schlickern übereinstimmt, die aber einen höheren Gehalt an Farbkomponenten aufweisen. Diese intensiv eingefärbten Schlicker sind vorzugsweise photoreaktiv und können durch Bestrahlung örtlich fixiert werden, bevor ggf. der Fügeschlicker appliziert wird und die Einzelteile der Zahnrestauration final zusammengefügt werden.

Anschließend unterwirft man den Grünling der Dentalrestauration in einem weiteren Schritt einer Wärmebehandlung zur Entfernung des Bindemittels (Entbinderung), um einen Braunling der Dentalrestauration zu erhalten. In einem nachfolgenden Schritt sintert man den Braunling der Dentalrestauration, um die fertige Dentalrestauration zu erhalten.

Die Herstellung der Grünlinge von Core- und Shellstruktur erfolgt vorzugsweise durch Stereolithographie. Hierbei werden durch schichtweise Strahlungshärtung von bei Verarbeitungstemperatur fließfähigen keramischen Schlickern keramische Grünlinge der Core- und der Shellstruktur hergestellt, die dann zum Grünling der Dentalrestauration zusammengefügt werden. Das Zusammenfügen kann händisch erfolgen. Vorzugsweis verwendet man zum Zusammenfügen der Grünlinge einen Fügeschlicker. Besonders bevorzugt verwendet man einen photoreaktiven Fügeschlicker und härtet diesen nach dem Fügen durch Bestrahlen mit Licht einer geeigneten Wellenlänge. Auf diese Weise erhöht man die Festigkeit des Grünlings der Dentalrestauration, erleichtert dessen Handhabung und verringert das Risiko von Beschädigungen.

Der Grünling der Dentalrestauration wird anschließend entbindert, vorzugsweise durch Erhitzen auf eine Temperatur von 90°C bis 600°C.

Der dabei erhaltene Braunling der Dentalrestauration wird dann zu einem dichten keramischen Formkörper gesintert. Die Sinterung des erfolgt vorzugsweise durch Erhitzen auf eine Temperatur von 650 bis 1800°C, beispielsweise in einem Sinterofen. Die Sinterbedingungen richten sich nach dem verwendeten Baumaterial. Braunlinge aus Glaskeramik werden vorzugsweise bei einer Temperatur von 650 bis 1100°C, besonders bevorzugt 700 bis 950°C, Braunlinge aus Zirkondioxid vorzugsweise bei 1100 bis 1600°C, besonders bevorzugt 1300 bis 1500°C, und Braunlinge aus Aluminiumoxid vorzugsweise bei 1400 bis 1800°C, besonders bevorzugt 1600 bis 1700°C gesintert.

Die nach dem erfindungsgemäßen Verfahren hergestellten Keramikformkörper zeichnen sich durch eine hohe Festigkeit und große Detailgenauigkeit aus. Die Biegefestigkeit nach ISO 6872 liegt bei Formkörpern aus Glaskeramik vorzugsweise über 100 MPa, insbesondere im Bereich von 150 bis 500 MPa. Formkörper aus Al₂O₃ haben eine Biegefestigkeit von vorzugsweise über 300 MPa, insbesondere von 500 bis 700 MPa, Formkörper aus ZrO₂ von mehr als 500 MPa insbesondere von 800 bis 1100 MPa.

Erfindungsgemäß werden zur Herstellung des Cores und der Shellstruktur und ggf. als Fügeschlicker Schlicker mit ähnlichen Wärmeausdehnungskoeffizienten und ähnlichem Sinterschrumpf verwendet, so dass der Grünkörper der Dentalrestauration problemlos entbindert und gesintert werden kann. Die Übereinstimmung von Wärmeausdehnungskoeffizienten und Sinterschrumpf ermöglicht es, die Grünkörper von Core und Shellstruktur passgenau herzustellen, weil kein unterschiedliches Schrumpfverhalten zu berücksichtigen ist. Das Vereinfacht das Zusammenfügen von Core und Shellstruktur erheblich und verringert das Fehlerrisiko, beispielsweise durch unbeabsichtigtes Verschieben der Shellstruktur auf dem Core.

Um das Zusammenfügen von Core und Shellstruktur zu erleichtern, können Core und Shellstruktur mit Einschubrillen, Fugen, Rippen oder ähnlichen Strukturen versehen werden, die Core und Shell ideal zueinander ausrichten und eine eindeutige, formschlüssige Ausrichtung der Formteile ermöglichen. Außerdem können solche Strukturen nach dem Sintern optische Funktionen übernehmen. Beispielsweise können sie die natürliche Mamelon-Struktur der Zähne nachahmen.

Die erfindungsgemäßen Dentalrestaurationen enthalten eine tragende Corestruktur und eine Shellstruktur. Die Shellstruktur wird an der Corestruktur befestigt. Die Corestruktur wird beim Einsetzen der fertigen Restauration im Munde des Patienten am zu restaurierenden Zahn bzw. Zähnen oder an einem Implantat bzw. Implantaten befestigt. Die Shellstruktur kann die Oberfläche des Cores ganz oder vorzugsweise teilweise umfassen bzw. abdecken. Die Shellstruktur der Dentalrestauration kann eine oder mehrere, vorzugsweise 1 oder 2, Komponenten enthalten, die zusammen die Shellstruktur bilden. Vorzugsweise enthält die Shellstruktur nur eine Komponente.

Die Verwendung von mehreren Komponenten ist aufwendiger, aber vorteilhaft, weil Corestrukturen mit Hinterschneidungen besser ummantelt werden können. Außerdem kann die Aufteilung der Shellstruktur in mehrere Komponenten herstellungstechnisch vorteilhaft sein. Beispielswese lassen sich zwei getrennte Komponenten zur Verblendung von Front und Rückseite einer Corestruktur leichter herstellen als eine einteilige Shellstruktur, die beide Bereiche abdeckt. Auch die Gefahr der Bildung von Spannungen und von Lufteinschlüssen beim Fügen kann durch die Verwendung mehrteiliger Shellstrukturen reduziert werden. Die Verwendung von mehreren Komponenten kann zudem ästhetische Vorteile haben. Beispielsweise können mehrere unterschiedliche gefärbte Shellstrukturen übereinandergelegt werden, um einen besonders natürlichen Eindruck zu erzielen.

Zur Herstellung der Core- und der Shellstruktur und als Fügeschlicker werden vorzugsweise Schlicker eingesetzt, die
(a) mindestens ein radikalisch polymerisierbares Monomer,
(b) mindestens einen Photoinitiator und
(c) Keramik-, Glas- und/oder Glaskeramikpartikel enthalten.

Die erfindungsgemäßen Schlicker enthalten als **Monomer (a)** mindestens ein (Meth)acrylat und/oder (Meth)acrylamid, vorzugsweise mono- oder multifunktionelle (Meth)acrylate oder eine Mischung davon. Besonders bevorzugt sind Werkstoffe, die als radikalisch polymerisierbares Monomer mindestens ein multifunktionelles (Meth)acrylat oder eine Mischung von mono- und multifunktionellen (Meth)acrylaten enthalten. Unter monofunktionellen (Meth)acrylaten werden Verbindungen mit einer, unter multifunktionellen (Meth)acrylaten Verbindungen mit zwei oder mehr, vorzugsweise 2 bis 6 radikalisch polymerisierbaren Gruppen verstanden.

Besonders bevorzugte mono- oder multifunktionelle (Meth)acrylate sind Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-G(M)A (ein Additionsprodukt aus (Meth)acrylsäure und Bisphenol-A-diglycidylether), ethoxy- oder propoxyliertes Bisphenol-A-di(meth)acrylat, wie z.B. Bisphenol-A-di(meth)acrylat mit 3 (SR-348C = Methacrylat; SR-349 = Acrylat, Fa. Sartomer) oder 2 Ethoxygruppen (SR-348L = Methacrylat, Fa. Sartomer), 2,2-Bis[4-(2-(meth)acryloxypropoxy)phenyl]propan, UD(M)A (ein Additionsprodukt aus 2-Hydroxyethyl(meth)acrylat und 2,2,4- oder 2,4,4-Trimethylhexamethylen-1,6-diisocyanat), Di-, Tri-, Tetra-, Penta-, Hexa- oder Hepta-ethylenglycoldi(meth)acrylat, Di-, Tri-, Tetra-, Penta-, Hexa- oder Hepta-propylenglycoldi(meth)acrylat, Trimethylolpropantri(meth)acrylat, Pentaerythrittetra(meth)acrylat, sowie Glycerindi- und -tri(meth)acrylat, 1,4-Butandioldi(meth)-acrylat, 1,10-Decandioldi(meth)acrylat (D₃MA), 1,12-Dodecandioldi(meth)acrylat, oligomere Polyether-, Polyester-, Epoxy-, Urethan-(meth)acrylate, Tricyclodecandimethanoldi(meth)acrylat und Mischungen davon.

Ganz besonders bevorzugt sind mono-, di-, trifunktionelle Acrylate und Methacrylate mit einem Molekulargewicht von <1000g/mol, wie z.B. aliphatische Urethandiacrylate, Phthalsäure-HEA-Ester (Photomer 4173), Pyromellitsäuredi-HEA-Ester (HEA = 2-Hydroxyethylacrylat), Bisphenol-A-di(meth)acrylat, Bis-G(M)A (ein Additionsprodukt aus (Meth)acrylsäure und Bisphenol-A-diglycidylether), ethoxy- oder propoxyliertes Bisphenol-A-di(meth)acrylat, wie Bisphenol-A-di(meth)acrylat (SR-348C, SR-349, SR-348L), 2,2-Bis[4-(2-(meth)acryloxyp-ropoxy)phenyl]propan, UD(M)A, Triethylenglycoldi(meth)acrylat (TEGD(M)A), Tricyclodecan-dimethanoldi(meth)acrylat, ethoxyliertes oder propoxyliertes Trimethylolpropantri(meth)acrylat, z.B. 3-fach propoxyliertes Trimethylolpropantriacrylat (Sartomer SR-492), Tripropylenglycoldiacrylat und Mischungen davon. Diese Monomere zeichnen sich durch eine hohe Reaktivität/einen hohen Doppelbindungsumsatz, gute mechanische Eigenschaften, einen geringen Polymerisationsschrumpf und eine relativ niedrige Viskosität aus.

Weiterhin geeignete Monomere sind Acrylamide wie N-Ethylacrylamid, N,N-Dimethylacrylamid, N-(2-Hydroxyethyl)acrylamid, N,N'-Diethyl-1,3-bis(acrylamido)-propan und 1,4-Bis-(acrylamido)-butan. Dabei werden die Bisacrylamide im Vergleich zu den Monoacrylamiden im organischen Bindemittel vorzugsweise im Überschuss eingesetzt.

Die Eigenschaften der Schlicker vor und nach der Lichthärtung lassen sich durch eine gezielte Kombinationen der Monomere beeinflussen. Mischungen aus monofunktionellen und difunktionellen Monomeren zeichnen sich durch eine relativ geringe Viskosität und Reaktivität der Harzmischung aus, wobei Viskosität und Reaktivität mit dem Gehalt an monofunktionellen Monomeren abnehmen. Ein Gehalt an monofunktionellen Monomeren gewährleistet eine geringere Sprödigkeit und ein schnellere Entbinderung der durch Lichthärtung der Schlicker erhaltenen Grünlinge. Mischungen aus difunktionellen und trifunktionellen Monomeren weisen eine höhere Reaktivität auf, wobei die Reaktivität mit dem Gehalt an trifunktionellen Monomeren zunimmt. Der Gehalt an trifunktionellen Monomeren bewirkt eine höhere Sprödigkeit und langsamere Entbinderung der Grünlinge.

Reaktivität und Viskosität der Harzmischung sowie der Polymerisationsschrumpf werden weiterhin durch die Molmasse der Monomeren bestimmt, wobei mit zunehmender Molmasse der Polymerisationsschrumpf abnimmt, während die Viskosität ansteigt. Schließlich kann über die Polarität der Monomeren die Wechselwirkung mit dem Werkstoff der stereolithographischen Wanne, wie z.B. die Quellung des Materials der Polymerisationswanne, beeinflusst werden.

Bevorzugte Photoinitiatoren (b) zur Initiierung der radikalischen Photopolymerisation sind Benzophenon, Benzoin sowie deren Derivate oder Diketone oder deren Derivate, wie 9,10-Phenanthrenchinon, 1-Phenyl-propan-1,2-dion, Diacetyl oder 4,4'-Dichlorbenzil. Besonders bevorzugt werden Campherchinon (CQ) und 2,2-Dimethoxy-2-phenyl-acetophenon und ganz besonders bevorzugt α-Diketone in Kombination mit Aminen als Reduktionsmittel eingesetzt, wie z.B. 4-(Dimethylamino)-benzoesäureester (EDMAB), N,N-Dimethylaminoethylmethacrylat, N,N-Dimethyl-sym.-xylidin oder Triethanolamin.

Besonders bevorzugte Photoinitiatoren sind Norrish-Typ-I-Photoinitiatoren, vor allem Monoacyl- oder Bisacylphosphinoxide, und insbesondere Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium (MBDEGe). Dabei lassen sich vorteilhaft auch Mischungen der verschiedenen Photoinitiatoren einsetzen, wie z.B. Bis(4-methoxybenzoyl)-diethylgermanium in Kombination mit Campherchinon und 4-Dimethylaminobenzoesäureethylester.

Ganz besonders bevorzugt sind Campherchinon (CAS Nr. 10373-78-1) in Kombination mit Ethyl-4-(dimethylamino)benzoat (EMBO, CAS Nr. 10287-53-3), sowie Norrish Typ I Photoinitiatoren, insbesondere 2,4,6-Trimethylbenzoyldiphenylphosphinoxid (TPO, CAS Nr. 75980-60-8), Ethyl(2,4,6-trimethylbenzoyl)phenylphosphinat (TPO-L, CAS Nr. 84434-11-7), Phenylbis(2,4,6-trimethylbenzoyl)phosphinoxid (Irgacure 819, CAS Nr. 162881-26-7), Bis(2,6-difluoro-3-(1-hydropyrrol-1-yl)phenyl)titanocen (Irgacure 784, CAS Nr. 125051-32-3), 2-Benzyl-2-(dimethylamino)-4'-morpholinobutyrophenon (Irgacure 369, CAS Nr. 119313-12-1), 1-Butanon-2-(dimethylamino)-2-(4-methylphenyl)methyl-1-4-(4-morpholinyl)phenyl (Irgacure 379, CAS-Nr. 119344-86-4) und ganz besonders Bis(4-methoxybenzoyl)diethylgermanium (MBDEGe; Ivocerin).

Als Komponente (c) enthalten die erfindungsgemäßen Schlicker Keramik- und/oder Glaspartikel und/oder Glaskeramikpartikel. Unter Keramiken werden anorganische Werkstoffe verstanden, die eine kristalline Struktur aufweisen und meist aus entsprechenden Pulvern hergestellt werden. Vorzugsweise erfolgt die Herstellung der Keramik durch Sintern (Sinterkeramik). Oxidkeramiken werden vorzugsweise durch Sintern von Metalloxidpulvern wie z.B. ZrO₂ oder Al₂O₃ erhalten.

Erfindungsgemäß sind Schlicker bevorzugt, die als Komponente (c) Glas- und/oder Glaskeramikpartikel enthalten. Bei Glaskeramiken handelt es sich um Werkstoffe, die meist aus amorphen Gläsern, insbesondere Silikatgläsern, durch gesteuerte Kristallisation hergestellt werden und bei denen eine Glasphase und eine oder mehrere Kristallphasen im Festkörper nebeneinander vorliegen. Besonders bevorzugt sind Glas- oder Glaskeramikpartikel, die Leucit-, Apatit- und ganz besonders bevorzugt Lithiumdisilikatkristalle enthalten. Glaskeramiken lassen sich aus entsprechend zusammengesetzten Glaspulvern, die keine Anteile an Kristalliten enthalten, durch thermische Behandlung (Kristallisations- und Sinterbrand) herstellen. Gemäß einer Ausführungsform können die erfindungsgemäßen Schlicker daher Glaspulver enthalten, die erst beim Sintern der Dentalrestauration in die entsprechende Glaskeramik übergehen.

Gemäß einer weiteren bevorzugten Ausführungsform können neben reinem Glas- bzw. Glaskeramikpulver auch Mischungen aus Glas- und Glaskeramikpulver verwendet werden. Bevorzugte Mischungen enthalten 0,5 bis 99,99 Gew.-% Glaskeramikpulver, bezogen auf die Gesamtmasse von Glas- und Glaskeramikpulver, wobei der Anteil des Glaspulvers vorzugsweise der Differenz zu 100 Gew.-% entspricht. Auf diese Weise können die Sinteraktivität und Formstabilität eingestellt werden. Gläser haben eine höhere Sinteraktivität (weniger Poren, glattere Oberfläche), während Glaskeramikpulver eine höhere Formstabilität (kein Verzug) zeigen. Außerdem sind Glaskeramikpulver im Schlicker chemisch stabiler gegen das Herauslösen von Ionen sowie die Bildung von Carbonaten und anderen unerwünschten Produkten und ergeben so stabilere Schlickereigenschaften.

Erfindungsgemäß bevorzugte Glaskeramiken werden in EP 1 505 041 A1, EP 2 261 184 A1, EP 2 377 830 A1 und EP 2 377 831 A1 im Einzelnen beschrieben. Besonders bevorzugt sind die in DE 103 36 913 A1 beschriebenen Lithiumsilikat-Materialien und ganz besonders bevorzugt die in EP 0 827 941 A1 beschriebenen Lithiumdisilikat-Glaskeramiken.

Die als Komponente (c) eingesetzten Keramik-, Glas- oder Glaskeramikpartikel sind vorzugsweise eingefärbt, und besonders bevorzugt werden zur Herstellung von Core- und der Shellstruktur jeweils unterschiedlich eingefärbte Schlicker verwendet. Um ein natürliches Aussehen der Dentalrestauration zu erzielen, das den durch Dentin und Zahnschmelz bedingten Kern-Schale-Eindruck des Zahns imitiert, wird der Schlicker zur Herstellung des Cores dunkler und opaker und der Schlicker zur Herstellung der Shellstruktur dem natürlichen Zahnschmelz entsprechend heller und transparenter eingefärbt.

Hierzu werden die Keramik-, Glas- und/oder Glaskeramikpartikel vorzugsweise mit einem oder mehreren Pigmenten gemischt. Besonders bevorzugt sind anorganische Pigmente, wie beispielsweise die Oxide von Pr, Tb, Ce, Co, Ni, Cu, Bi, La, Nd, Sm, Eu, Gd und insbesondere von Fe, Mn, Cr, Er. Zur Einfärbung der Keramik- oder Glaskeramikpulver können vorteilhaft auch farbige Spinell-Verbindungen des Typs AB₂O₄ verwendet werden, wobei A bevorzugt ein Alkali- oder Erdalkalimetallion und B ein Übergangsmetallion höherer Oxidationsstufe als A ist. Die Spinelle eignen sich besonders zur Einfärbung von Glaskeramik und insbesondere von Lithiumdisilikatglaskeramik. Die farbgebenden Komponenten werden einer Menge zugesetzt, die erforderlich ist, um die gewünschte Färbung zu erzielen. Vorzugsweise werden Art und Menge der farbgebenden Komponente(n) so gewählt, dass nach dem Entbindern und Sintern zahnfarbene Keramikformteile erhalten werden. Der Farbeindruck wird erst während des Sinterns endgültig ausgebildet. Üblicherweise werden die farbgebenden Komponenten in einer Menge von jeweils 0,01 bis 1 Gew.-%, bevorzugt 0,05 bis 0,5 Gew.-% bezogen auf die Gesamtmasse der Komponente (c) eingesetzt. Alternativ können die Keramik-, Glas- oder Glaskeramikpulver auch entsprechend EP 0 827 941 A1 durch die Zugabe glasfärbender Oxide (sog. lonenfärbung) eingefärbt werden. Bevorzugte glasfärbende Oxide sind TiO₂, CeO₂ und/oder Fe₂O₃.

Zur Erzielung des gewünschten Farbeindrucks ist es auch möglich, Mischungen von unterschiedlich eingefärbten Keramik-, Glas- oder Glaskeramikpulvern einzusetzen.

Die Partikelgröße der Komponente (c) liegt vorzugsweise im Bereich von 10 nm bis 100 µm, vorzugsweise 100 nm bis 10 µm. Sie ist abhängig von der eingesetzten Keramik. Bei Al₂O₃ liegt die Größe der als Komponente (c) verwendeten Partikel vorzugsweise im Bereich von 50 bis 500 nm, besonders bevorzugt zwischen 75 und 200 nm; bei Glaspulver und/oder Glaskeramikpulvern im Bereich von 500 nm bis 50 µm, ganz bevorzugt zwischen 1 und 15 µm; bei TZP-3Y Zirkondioxid im Bereich von 50 bis 500 nm, ganz bevorzugt zwischen 50 und 350 nm. Die Partikelgröße wird dabei vorzugsweise so gewählt, dass sedimentationsstabile Schlicker erhalten werden. Bei den angegebenen Unter- bzw. Obergrenzen der Partikelgrößenbereiche handelt es sich jeweils um die D10- bzw. D90-Werte. Ein Bereich von 500 nm bis 50 µm bedeutet also, dass der D10-Wert 500 nm und der D90-Wert 50 µm beträgt, d.h., 10 Vol.-% der Partikel sind kleiner als 500 nm und 90 Vol.-% der Partikel sind kleiner als 50 µm.

Weiterhin lassen sich auch Keramik-, Glas- oder Glaskeramikpartikel mit einer Partikelgröße im Bereich von 10 bis 200 nm als Nano- oder Organosole, d.h. als Dispersion der Nanopartikel in einem Lösungsmittel, einem geeigneten Monomer der Komponente (a) oder einer Mischung daraus einsetzen. Auch hier handelt es sich um den D10- bzw. D90-Wert.

Die Partikelgrößenbestimmung im Bereich von 0,1 µm bis 1000 µm erfolgt vorzugsweise mittels statischer Lichtstreuung (SLS), beispielsweise mit einem statischen Laserstreuungs-Partikelgrößen-Analysator LA-960 (Horiba, Japan) oder mit einem Microtrac S100 Partikelgrößen-Analysator (Microtrac, USA). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320. Die Messung der Partikelgröße in einem Bereich von 1 nm bis 0,1 µm erfolgt vorzugsweise durch dynamische Lichtstreuung (DLS) von wässrigen Partikeldispersionen, vorzugsweise mit einem He-Ne-Laser mit einer Wellenlänge von 633 nm, bei einem Streuwinkel von 90° und bei 25 °C, z.B. mit einem Malvern Zetasizer Nano ZS (Malvern Instruments, Malvern UK).

Im Fall aggregierter und agglomerierter Partikel kann die Primarteilchengröße anhand von TEM-Aufnahmen bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf ein 50 Å dickes Kupfergitter (Maschenweite 300 Mesh) aufgebracht, das mit Kohlenstoff beschichtet ist, und im Anschluss das Lösungsmittel verdampft. Die Partikel werden ausgezählt und der arithmetische Mittelwert berechnet.

Erfindungsgemäß besonders bevorzugt sind Gläser und Lithiumdisilikat-Glaskeramiken mit der folgenden Zusammensetzung:

| | |
|---|---|
| SiO₂ | 57,0 bis 80,0 Gew.-% |
| Al₂O₃ | 0 bis 5,0 Gew.-% |
| La₂O₃ | 0,1 bis 6,0 Gew.-% |
| MgO | 0 bis 5,0 Gew.-%, insbesondere 0,1 bis 5,0 Gew.-% |
| ZnO | 0 bis 8,0 Gew.-% |
| K₂O | 0 bis 13,5 Gew.-% |
| Li₂O | 11,0 bis 19,0 Gew.-% |
| P₂O₅ | 0 bis 11,0 Gew.-% |
| Farbkomponenten | 0 bis 8,0 Gew.-% |
| Zusatzkomponenten | 0 bis 6,0 Gew.-% |

wobei

| | |
|---|---|
| Al₂O₃ + La₂O₃ | 0,1 bis 7,0 Gew.-% und |
| MgO + ZnO | 0,1 bis 9,0 Gew.-% |

ausmachen und wobei die Farbkomponenten aus glasfärbenden Oxiden und/oder Farbkörpern in den folgenden Mengen gebildet sind:

| | |
|---|---|
| glasfärbende Oxide | 0 bis 5,0 Gew.-% und |
| Farbkörper | 0 bis 5,0 Gew.-%. |

Bevorzugte glasfärbende Oxide sind TiO₂, CeO₂ und/oder Fe₂O₃. Als Farbkörper sind die bei dentalen Glaskeramiken üblichen Metalloxide, wie die oben genannten anorganischen Pigmente, und insbesondere handelsübliche isochrome Farbkörper, wie z.B. farbige bzw. dotierte Spinelle und/oder dotiertes ZrO₂, bevorzugt. Bei den Farbkörpern kann es sich sowohl um nicht-fluoreszierende als auch fluoreszierende Materialien handeln. Wenn nicht anders angegeben, beziehen sich alle Prozentangaben hierin auf die Gesamtmasse der Glaskeramik.

Für die einzelnen Komponenten des erfindungsgemäß bevorzugten Glases bzw. Lithiumdisilikat-Glaskeramik existieren bevorzugte Mengenbereiche. Diese können unabhängig voneinander gewählt werden und sind wie folgt:

| | |
|---|---|
| SiO₂ | 57,0 bis 75,0 Gew.-% |
| Al₂O₃ | 0 bis 2,5 Gew.-% |
| La₂O₃ | 0,1 bis 4,0 Gew.-% |
| MgO | 0,1 bis 4,0 Gew.-% |
| ZnO | 0 bis 6,0 Gew.-%, insbesondere 0,1 bis 5,0 Gew.-% |
| K₂O | 0 bis 9,0 Gew.-%, insbesondere 0,5 bis 7,0 Gew.-% |
| Li₂O | 13,0 bis 19,0 Gew.-% |
| P₂O₅ | 0 bis 8,0 Gew.-%, insbesondere 0,5 bis 8,0 Gew.-% |
| Farbkomponenten | 0,05 bis 6,0 Gew.-% |
| Zusatzkomponenten | 0 bis 3,0 Gew.-%. |

Neben den genannten Komponenten kann das Glas bzw. die Lithiumdisilikat-Glaskeramik weitere Zusatzkomponenten enthalten, insbesondere B₂O₃, F, Na₂O, ZrO₂, BaO und/oder SrO. Dabei kann mit B₂O₃ und F die Viskosität der Restglasphase der Glaskeramik beeinflusst werden, und man nimmt an, dass sie das Verhältnis Oberflächen- zu Volumenkristallisation zugunsten der Oberflächenkristallisation verschieben.

Vorzugsweise besteht das Glas bzw. die Lithiumdisilikat-Glaskeramik im wesentlichen aus den zuvor genannten Komponenten.

Zur Herstellung des Glases bzw. der Lithiumdisilikat-Glaskeramik wird ein Ausgangsglas, das die genannten Komponenten mit Ausnahme der Farbkörper enthält, bei Temperaturen von 1200 bis 1650°C erschmolzen. Hierzu werden geeignete Ausgangsmaterialien, wie zum Beispiel Carbonate, Oxide und Fluoride, innig miteinander vermischt und auf die angegebenen Temperaturen erwärmt. Sofern farbgebende Oxide eingesetzt werden sollen, werden diese dem Gemenge zugegeben. Die erhaltende Glasschmelze wird unter Bildung eines Glasgranulats in Wasser eingegossen und das Glasgranulat dann zu einem Pulver mit der gewünschten Korngröße zerkleinert. Anschließend werden dem Pulver die ggf. vorhandenen Farbkörper zugesetzt. Dieses Glaspulver kann dann zur Herstellung der gewünschten Schlicker verwendet werden. In diesem Fall erfolgt die Bildung einer Glaskeramik während des Sinterns. Vorzugsweise wird das Glaspulver einer Wärmebehandlung im Temperaturbereich von 400 bis 1100°C unterzogen. Die Wärmebehandlung dient zu Auslösung der Kristallisation des Ausgangsglases und damit zur Bildung der Glaskeramik, die nach Abschluss der Wärmebehandlung vorliegt und die dann zur Herstellung eines Schlickers eingesetzt werden kann. Wie zuvor erwähnt, können auch Mischungen von Glas- und Glaskeramikpulver zur Herstellung der Schlicker verwendet werden.

Die erfindungsgemäß bevorzugten Gläser und Lithiumdisilikat-Glaskeramiken sowie deren Herstellung werden in EP 0 827 941 A1 detailliert beschrieben, wobei die dort konkret beschriebenen Glaskeramiken besonders bevorzugt sind.

Gemäß einer bevorzugen Ausführungsform der Erfindung werden die Partikel der Komponente (c) mit geeigneten Stoffen oberflächenmodifiziert. Zur Oberflächenmodifizierung werden vorzugsweise solche Verbindungen eingesetzt, die chemisch, d.h. durch ionische oder kovalente Bindungen an die Oberfläche der Keramik-, Glas- und/ oder Glaskeramikpartikel gebunden werden. Bevorzugt sind Verbindungen, die entweder Säuregruppen, vorzugsweise Carbonsäure-, Phosphonsäure-, Hydrogenphosphatgruppen oder saure Phosphorsäureestergruppen, oder Silylgruppen, vorzugsweise Alkoxysilylgruppen enthalten. Die Partikeloberfläche kann teilweise oder vorzugsweise vollständig mit dem Modifizierungsmittel bedeckt sein. Bei den erfindungsgemäß verwendeten Modifizierungsmitteln handelt es sich um monomere Verbindungen.

Erfindungsgemäß sind solche Verbindungen besonders geeignet, die im Gegensatz zu den sogenannten Haftvermittlern oder Kopplungsreagenzien nur mit der Partikeloberfläche reagierende Gruppen enthalten, aber keine radikalisch polymerisierbaren Gruppen, die mit der Harzmatrix (a) eine kovalente Bindung eingehen. Solche Verbindungen werden hierin als nicht polymerisierbare Oberflächenmodifizierungsmittel bezeichnet. Diese Verbindungen haben den Vorteil, dass ein stabiler Verbund zwischen der Partikeloberfläche und der Polymermatrix im ausgehärteten Grünling nicht zustande kommt, was die vollständige Entfernung der Polymeranteile im Entbinderungsprozess vereinfacht.

Als nicht polymerisierbare Oberflächenmodifizierungsmittel eignen sich insbesondere lineare oder verzweigte Carbonsäuren, wie z.B. Ameisensäure, Essigsäure, Propionsäure, Octansäure, Isobuttersäure, Isovaleriansäure, Pivalinsäure oder Phosphonsäuren, z.B. wie Methyl-, Ethyl-, Propyl-, Butyl-, Hexyl-, Octyl- oder Phenylphosphonsäure. Als nicht polymerisierbare Oberflächenmodifikatoren geeignete Silane sind beispielsweise Propyltrimethoxysilan, Phenyltrimethoxysilan, Hexyltrimethoxysilan, Octyltrimethoxysilan, Trimethylchlorsilan, Trimethylbromsilan, Trimethylmethoxysilan oder Hexamethyldisilazan. Ganz besonders bevorzugt sind saure Phosphorsäureester, wie z.B. Dimethyl-, Diethyl-, Dipropyl-, Dibutyl-, Dipentyl-, Dihexyl-, Dioctyl- oder Di(2-ethylhexyl)phosphat.

Als Oberflächenmodifizierungsmittel eignen sich für wässrige Schlicker weiterhin Polymere, insbesondere Polyelektrolyte, z.B. Polycarbonsäuren oder Polycarbonsäuresalze, oder nichtionische Polymere, wie z.B. Polyethylenglycol oder Carboxymethylcellulose. Polyelektrolyte, die wie z.B. Ammoniumpolycarboxylat ionische Gruppen tragen, können relativ leicht an die Oberfläche von Festkörpern adsorbieren und den Partikeln dabei eine elektrische Ladung verleihen. Im Fall von organischen, nicht wässrigen Schlickern eignen sich Polymere, die im Polyreaktionsharz löslich sind. Modifizierungsmittel mit einer Molmasse im Bereich von 50 bis 5.000 g/mol sind bevorzugt.

Oberflächenmodifizierungsmittel werden ggf. in einer Menge von vorzugsweise 0,1 bis 5 Gew.- %, besonders bevorzugt 0,2 bis 2 Gew.-% und ganz besonders bevorzugt 0,5 bis 1,5 Gew.-% bezogen auf die Masse des Schlickers eingesetzt.

Die erfindungsgemäßen Schlicker können als Komponente **(d)** ein oder mehrere nichtionische Tenside enthalten. Nichtionische Tenside sind Stoffe mit grenzflächenaktiven Eigenschaften, die in wässrigen Medien keine Ionen bilden. Es handelt sich um Moleküle, die einen hydrophoben und einen hydrophilen Teil aufweisen. Durch Wahl der Länge und Art der hydrophoben und hydrophilen Teile kann die Gesamthydrophobizität der Moleküle eingestellt werden.

Erfindungsgemäß sind nichtionische Tenside mit einem HLB-Wert im Bereich von 3 bis 16, insbesondere 4 bis 13 und ganz besonders von 4 bis 10 bevorzugt. Der HLB-Wert wird nach der Methode von Griffin bestimmt.

Bevorzugte nichtionische Tenside (d) sind die Ethoxylate von Fettalkoholen, Oxoalkoholen oder Fettsäuren, Fettsäureester von Zuckern und hydrierten Zuckern, Alkylglycoside sowie Blockpolymere des Ethylen- und Propylenoxids, insbesondere kurzkettige Block-Co-Oligomere.

Besonders bevorzugt sind Fettsäureester von hydrierten Zuckern, insbesondere solche mit der Formel R'-CO-O-Zucker, wobei R' ein verzweigter oder bevorzugt gerader Alkylrest mit 10 bis 25 Kohlenstoffatomen, vorzugsweise 12 bis 22 Kohlenstoffatomen ist. Besonders bevorzugt sind gerade Alkylreste mit 16 bis 22 Kohlenstoffatomen. "Zucker" steht für einen hydrierten Zuckerrest, der vorzugsweise 1- bis 5-fach ethoxyliert ist. Ganz besonders bevorzugt sind Fettsäureester des Sorbitols, insbesondere Sorbitanstearate wie z.B. Sorbitanmonostearat (CAS 1338-41-6).

Eine weitere besonders bevorzugte Gruppe von Tensiden sind Ethoxylate von Fettsäuren, insbesondere solche mit der allgemeinen Formel R"-(CO)-(OCH₂CH₂)ₘ-OH, in der R" ein verzweigter oder bevorzugt gerader Alkylrest mit 10 bis 25 Kohlenstoffatomen, vorzugsweise 12 bis 22 Kohlenstoffatomen ist. Besonders bevorzugt sind gerade Alkylreste mit 16 bis 22 Kohlenstoffatomen. m ist eine ganze Zahl von 2 bis 20, bevorzugt 2 bis 10 und besonders bevorzugt 2 bis 6.

Erfindungsgemäß ganz besonders bevorzugte Tenside (d) sind Ethoxylate von Fettalkoholen, insbesondere Polyalkylenglycolether mit der allgemeinen Formel R-(OCH₂CH₂)ₙ-OH, in der R ein Alkylrest mit 10 bis 20 Kohlenstoffatomen und n eine ganze Zahl von 2 bis 25 ist. R kann ein verzweigter oder bevorzugt gerader Alkylrest sein, wobei Alkylreste mit 12 bis 22 Kohlenstoffatomen und besonders gerade Alkylreste mit 12 bis 22 Kohlenstoffatomen bevorzugt sind. Ganz besonders bevorzugte Alkylreste sind Lauryl, Cetyl, Cetearyl und Stearyl.

Die Polyalkylenglycolether sind durch Umsetzung der entsprechenden Fettalkohole mit Ethylenoxid (EO) erhältlich. Der Index n gibt die Anzahl an Ethylenoxidresten an. Polyalkylenglycolether mit 2 bis 21 (n = 2-21), insbesondere 2 bis 12 (n = 2-12) und ganz besonders 2 bis 5 (n = 2-5) Ethylenoxidresten sind bevorzugt.

Beispiele für erfindungsgemäß bevorzugte Polyalkylenglycolether sind Verbindungen, in denen R ein Cetylrest (C₁₆-Rest) und n 20 und insbesondere 2 ist. Diese Verbindungen haben die INCI-Bezeichnungen Ceteth-2 und Ceteth-20. Ceteth-2 hat z.B. die Formel C₁₆H₃₃-(OCH₂CH₂)₂-OH.

Weiter bevorzugt sind Verbindungen, in denen R ein Stearylrest (C₁₈-Rest) und n 2, 10, 20 oder 21 ist. Diese Verbindungen haben die INCI-Bezeichnungen Steareth-2, Steareth-10, Steareth-20 und Steareth-21. Steareth-2 hat z.B. die Formel C₁₈H₃₇-(OCH₂CH₂)₂-OH.

Ganz besonders bevorzugte nichtionische Tenside sind Steareth-20 (HLB = 15,3), Steareth-10 (HLB = 12,4), Ceteth-20 (HLB = 12,9) und insbesondere Steareth-2 (HLB = 4,9) und Ceteth-2 (HLB = 5,3).

Mischungen unterschiedlicher nichtionischer Tenside und insbesondere unterschiedlicher Polyalkylenglycolether können ebenfalls verwendet werden.

INCI steht für *International Nomenclature of Cosmetic Ingredients.* Es handelt sich um eine internationale Richtlinie für die korrekte Angabe von Inhaltsstoffen von Kosmetika. Erfindungsgemäß wurde völlig überraschend gefunden, dass die Verwendung von nichtionischen Tensiden, die üblicherweise zur Herstellung von kosmetischen und pharmazeutischen Produkten eingesetzt werden, die Eigenschaften von Schlickern für die stereolithographische Herstellung von Keramik- und Glaskeramikkörpern deutlich verbessern.

Es wurde gefunden, dass die erfindungsgemäß verwendeten nichtionischen Tenside mit radikalisch polymerisierbaren Monomeren und insbesondere mit Acrylaten und Methacrylaten homogen mischbar sind und die Polymerisation dieser Monomere nicht beeinträchtigen. Bei der stereolithographischen Verarbeitung der erfindungsgemäßen Schlicker im flüssigen Zustand können dadurch eine hohe Reaktivität sowie kurze Belichtungs- und Prozesszeiten gewährleistet werden. Vorteilhaft ist, dass die nichtionischen Tenside auch mit kurzkettigen polaren und stark reaktiven Monomeren gut mischbar sind.

Die erfindungsgemäßen nichtionischen Tenside sind vorzugsweise bei einer Temperatur von < 20°C und besonders bevorzugt < 30°C fest. Dies hat zur Folge, dass sie zu einer Verfestigung der Schlicker führen, d.h. die Schlicker haben bei einer Temperatur von < 20°C und vorzugsweise < 30°C eine pastenförmige bis feste Konsistenz. Ein Vorteil davon ist, dass durch die homogene Erstarrung eine Sedimentation der Partikel verhindert wird und dadurch die Lagerstabilität bei Lagerung bei Raumtemperatur im festen Zustand deutlich erhöht ist. Außerdem verbessern die nichtionischen Tenside die Festigkeit der polymerisierten Objekte (Grünlinge) bei Raumtemperatur im Vergleich zu Grünlingen aus Schlickern ohne den Bestandteil (d).

Bevorzugt sind Schlicker, die als nichtionisches Tensid mit einem Schmelzpunkt von 20°C bis 120°C, bevorzugt 20°C bis 100°C, besonders bevorzugt 20°C bis 60°C enthalten. Die Schlickerkomponenten sind oberhalb des Schmelzpunktes von Komponente (d) homogen mischbar und erstarren unterhalb des Schmelzpunktes von (d) homogen. Eine Sedimentation der Partikel wird damit unterhalb des Schmelzpunkts der Komponente (d) wirksam verhindert. Dadurch ist die Stabilität der Mischung über lange Zeit gewährleistet.

Besonders vorteilhaft ist, dass die erfindungsgemäß verwendeten nichtionischen Tenside auch im flüssigen Zustand wirksam eine vorzeitige Sedimentation der Partikel verhindern, woraus eine hohe Stabilität der Schlicker bei der Verarbeitung resultiert.

Durch die guten Dispergiereigenschaften der nichtionischen Tenside für Partikel können die Schlicker zudem zu einem signifikant höheren Grad mit Keramik-, Glas- und/oder Glaskeramikpulver gefüllt werden, was in einer höheren Gründichte resultiert.

Ein weiterer Vorteil der erfindungsgemäß bevorzugten nichtionischen Tenside (d) ist, dass diese beim Entbindern schmelzen und wegen ihres relativ geringen Molekulargewichte aus der Matrix heraus fließen oder abdampfen. Sie behindern das Entbindern nicht, sondern schaffen vielmehr Kanäle, die den Austritt der Zersetzungsprodukte der organischen Matrix begünstigen und so das Entbindern erleichtern. Beim Aufheizvorgang im Ofen fließen die zwischen den Polymerketten der Matrix befindlichen Moleküle des nichtionischen Tensids (d) in flüssiger Form aus dem Grünkörper aus, bevor die polymerisierten Anteile der Mischung depolymerisiert und dann pyrolysiert werden. Das Risiko von Defekten wie Rissen oder gar der Zerstörung des Formkörpers während des Entbinderungsvorgangs wird dadurch zumindest stark reduziert und meist ganz eliminiert. Zudem wird durch das Ausfliessen/Abdampfen von Komponente (d) bereits ein Teil der organischen Masse bei vergleichsweise niedriger Temperatur entfernt, und der weitere Aufheizvorgang bis zur vollständigen Entfernung aller organischen Anteile kann zügig durchgeführt werden, während bisher nur relativ geringe Aufheizraten möglich waren.

Neben den oben genannten Komponenten enthalten die erfindungsgemäßen Schlicker vorzugsweise zusätzlich mindestens ein **Additiv,** das aus Farbstoffen, UV-Absorbern, optischen Aufhellern, Lösungsmitteln, Inhibitoren, Entbinderungsbeschleunigern, Entschäumungsmitteln und/oder Hautverhinderungsmitteln ausgewählt ist.

Als **Farbstoff** sind organische Farbstoffe bevorzugt, insbesondere Azofarbstoffe, Carbonylfarbstoffe, Cyaninfarbstoffe, Azomethine und Methine, Phthalocyanine und Dioxazine. Besonders bevorzugt sind Farbstoffe, die in dem Schlicker löslich sind, insbesondere Azofarbstoffe. Ein ganz besonders bevorzugter Farbstoff ist (4-(4-Nitrophenylazo)anilin (Disperse Orange 3, CAS Nr. 730-40-5). Besonders bevorzugt sind weiterhin Farbstoffe, die im gleichen Wellenlängenbereich absorbieren wie der Polymerisationsinitiator. Besonders bevorzugt sind Farbstoffe, die ein Absorptionsmaximum aufweisen, das der Wellenlänge des zur Härtung eingesetzten Lichts entspricht. Sehr vorteilhaft sind Farbstoffe mit einem Absorptionsmaximum im Bereich von 350 bis 550 nm, vorzugsweise 380 bis 480 nm.

Anders als bei den zur Einfärbung der Keramik- bzw. Glaskeramikpulver verwendeten farbgebenden Komponenten werden unter Farbstoffen hier solche Stoffe verstanden, die beim Entbindern und Sintern vollständig verbrennen. Diese Farbstoffe dienen allein dazu, den Schlicker einzufärben und so dessen Verarbeitung zu erleichtern. Sie dienen nicht zur Einfärbung der Keramik. Die Einfärbung kann hilfreich sein, um Schlicker einfacher voneinander unterscheiden zu können. Zudem absorbieren Farbstoffe während des stereolithographischen Druckens das eindringende Licht und verringern dessen Eindringtiefe in das Material und ermöglichen es so Objekte präziser zu drucken, was besonders bei sehr transparenten Schlickern vorteilhaft ist.

Bevorzugte UV-Absorber sind 2,2'-Methylenebis[6-(2H-benzotriazol-2-yl)-4-(1,1,3,3-tetramethylbutyl)phenol], 2,2',4,4'-Tetrahydroxybenzophenone, 2-tert-Butyl-6-(5-chloro-2H-benzotriazol-2-yl)-4-methylphenol, 2,2'-Benzene-1,4-diylbis(4h-3,1-benzoxazin-4-one, 2-(4,6-Bis-(2,4-dimethylphenyl)-1,3,5-triazin-2-yl)-5-(octyloxy)-phenol, 2-(2-Hydroxy-5-methylphenyl)benzo-triazole und 2-(2H-Benzotriazol-2-yl)-6-dodecyl-4-methylphenol. Ein erfindungsgemäß bevorzugter optische Aufheller ist 2,5-Bis(5-tert-butyl-benzoxazol-2-yl)thiophen. Besonders bevorzugt sind UV-Absorber und optische Aufheller, die ein Absorptionsmaximum aufweisen, das der Wellenlänge des zur Härtung eingesetzten Lichts entspricht.

Als **Lösungsmittel** sind organische Lösungsmittel bevorzugt, insbesondere solche Lösungsmittel, die eine Siedetemperatur von mindestens ca. 120°C, vorzugsweise von 150 bis 250°C aufweisen, so dass es bei der stereolithographischen Verarbeitung des Schlickers nicht zu einer vorzeitigen Verdunstung kommt. Dabei sind Mischungen von solchen Lösungsmitteln besonders geeignet, die in einem Temperaturbereich zwischen 150 und 250 °C schrittweise verdampfbar sind. Ganz besonders geeignet sind n-Octanol, Triethylenglycoldivinylether, 2-Amino-2-methyl-1-propanol, 2-Methyl-2,4-pentandiol, Tripropylenglycol, Tetraethylenglycol, Triethylenglycol, Triethylcitrat, Acetessigsäureethylester, Cyclohexanol, Cyclohexanon, Diethylenglycolmonomethylether, Oxalsäuredibutylester, 2,5-Dimethoxytetrahydrofuran, Polythylenlycol 300, 1- oder 2-Nonanol, Diethylenglycoldiethylether und Mischungen davon.

Bevorzugte Lösungsmittel sind außerdem Polyethylenglykole (PEG), Polypropylenglykole (PPG), PEG-PPG-Co-Polymere, Glycerin und Glycerinderivate, d.h. insbesondere ethoxyliertes und/oder propoxyliertes Glycerin, sowie Phthalate, Benzoate und cycloaliphatische Esther. Besonders bevorzugt sind Lösungsmittel mit einem Mw kleiner als 5.000 g/mol, bevorzugt kleiner als 1.000 g/mol. Als Lösungsmittel eignen sich Verbindungen, die bei Raumtemperatur flüssig sind und die mit den übrigen Komponenten des Schlickers unter Lager- und Anwendungsbedingungen nicht reagieren. Besonders bevorzugte Lösungsmittel sind PEG 200-600g/mol, PPG 200-800g/mol, Co-PEG-PPG 200-800g/mol und insbesondere Polypropylenglycol -400g/mol, Di-Ethyl-Phthalat, Di-Methyl-Phthalat, Ethyl-Benzoat, Butyl-Benzoat, Benzyl-Benzoat, Diethylenglykol-Dibenzoat, 1,2-Cyclohexandicarbonsäurediisononylester. Es können vorteilhaft auch Mischungen dieser Lösungsmittel eingesetzt werden.

Das oder die Lösungsmittel werden vorzugsweise in einer Gesamtmenge von 0 bis 50 Gew.-%, besonders bevorzugt von 3 bis 20 Gew.-% eingesetzt, bezogen auf die Masse des Schlickers.

Es wurde gefunden, dass das Verdampfen der obigen Lösungsmittel die Bildung von Mikroporen im Grünling zusätzlich fördert. Diese Poren schließen sich ebenso wie die durch das Ausfließen oder Abdampfen der nichtionischen Tenside gebildeten Kanäle beim Sintern. Sie erleichtern das Entweichen der Gase beim Entbindern und verhindern so das Entstehen von Spannungen und Rissen. Außerdem wird die Gefahr einer Trennung der stereolithographisch erzeugten Schichten reduziert und eine vollständige Entfernung aller organischen Komponenten begünstigt.

Alternativ kann die Porosität des Grünlings auch dadurch vergrößert werden, dass vor der Wärmebehandlung herauswaschbare Anteile extraktiv entfernt werden. Geeignete **extrahierbare Komponenten** müssen auf das Extraktionsmittel abgestimmt sein. Bei oxid-keramischen Partikeln kann Wasser als Extraktionsmittel verwendet werden. In diesem Fall sind wasserlösliche Polymere, wie z.B. Polyvinylalkohol, Polyvinylpyrrolidon und Polyethylenglycole geeignet. Bei Keramik-, Glas- und Glaskeramik-Partikeln, die empfindlich auf Wasser sind, können organische Lösungsmittel (besonders apolare Lösungsmittel) zur Extraktion verwendet werden. In diesem Fall eignen sich z.B. benzinlösliche Stoffe wie langkettige Fettsäureester als extrahierbare Komponenten. Die bevorzugte Menge an extrahierbaren Komponenten liegt zwischen 0 und 30 Gew.-%, besonders bevorzugt zwischen 1 und 20 Gew.-%, bezogen auf die Masse des Schlickers.

Die erfindungsgemäßen Schlicker können vorteilhaft als weiteres Additiv einen **Inhibitor** zur Verhinderung einer spontanen Polyreaktion als Stabilisator enthalten. Die Inhibitoren bzw. Stabilisatoren verbessern die Lagerstabilität der Schlicker und verhindern darüber hinaus eine unkontrollierte Polyreaktion in der stereolithographischen Wanne. Die Inhibitoren werden vorzugsweise in einer solchen Menge zugesetzt, dass die Schlicker über einen Zeitraum von ca. 2 bis 3 Jahre lagerstabil sind. Besonders bevorzugt werden die Inhibitoren in einer Menge von 0,001 bis 1,0 Gew.-%, ganz besonders bevorzugt 0,001 bis 0,50 Gew.-% eingesetzt, jeweils bezogen auf die Gesamtmasse des Schlickers.

Bevorzugt sind sog. aerobe Inhibitoren, z.B. Phenole, wie Hydrochinonmonomethylether (MEHQ) oder 2,6-Di-tert.-butyl-4-methyl-phenol (BHT), die nur in Gegenwart von Sauerstoff effektiv wirksam sind und vorzugsweise in einem Konzentrationsbereich von 100 bis 2000 ppmw verwendet werden. Geeignete anaerobe Inhibitoren sind Phenothiazin, 2,2,6,6-Tetramethyl-piperindin-1-oxyl-Radikal (TEMPO), lod und Kupfer-(I)-iodid. Diese wirken schon in geringen Konzentrationen von vorzugsweise 10 - 50 ppm auch bei Abwesenheit von Sauerstoff. Eine Polymerisation findet erst dann statt, wenn diese Additive verbraucht sind. Dabei ist es von Vorteil, eine Mischung von aeroben und anaeroben Inhibitoren einzusetzen.

Aerobe Inhibitoren werden vorzugsweise in einer Menge von 0,001 bis 0,50 Gew.-% und anaerobe Inhibitoren in einer Menge von 0,001 bis 0,02 Gew.-% eingesetzt, jeweils bezogen auf die Gesamtmasse des Schlickers. Bevorzugte Mischungen enthalten 0,005 bis 0,10 Gew.-% an aeroben Inhibitoren und 0,001 bis 0,01 Gew.-% an anaeroben Inhibitoren, ebenfalls bezogen auf die Gesamtmasse des Schlickers.

Gemäß einer weiteren Ausführungsform der Erfindung enthalten die Schlicker als zusätzliches Additiv einen sogenannten **Entbinderungsbeschleuniger.** Dieser wird vorzugsweise in einer Menge von 0 bis 20 Gew.-%, besonders bevorzugt 0,01 bis 10 Gew.-% eingesetzt, jeweils bezogen auf die Gesamtmasse des Schlickers. Unter Entbindungsbeschleunigern werden Stoffe verstanden, welche das Entfernen des Bindemittels während des Entbinderungsprozesses erleichtern.

Die Entbinderung des Grünlings kann z.B. durch polyreaktionswirksame Stoffe im Polyreaktionsharz gefördert oder zielgerichtet beeinflusst werden. Dabei handelt es sich einerseits um Additive, die die Netzwerkbildung beeinflussen, wie vor allem kettenübertragungsaktive Stoffe, sog. Kettenregler, die zu einer Verringerung der Polymernetzwerkdichte und damit zu einer besseren thermischen Abbaubarkeit führen. Bekannte Kettenregler z.B. für die radikalische Polymerisation sind vor allem Mercaptane, wie z.B. Laurylmercaptan, und Disulfide. Disulfide, vor allem Dithiourethandisulfide, wie z.B. Tetramethylthiuramdisulfid oder Isopropylxanthogensäuredisulfid wirken bei der radikalischen Photopolymerisation als sog. Photoiniferter. Es handelt sich dabei um Verbindungen, die sowohl als Photoinitiator (Photoini-) wirken, als auch an Übertragungsreaktionen (engl.: transfer, -fer-) und Abbruchsreaktionen (engl.: termination, - ter) teilnehmen (vgl. vgl. T. Ostsu, M. Yoshida, Makromol. Chem., Rapid. Commun. 3 (1982) 127-132: Role of Initiator-TransferAgent-Terminator (Iniferter) in Radical Polymerizations: Polymer Design by Organic Disulfides as Iniferters). Die Zugabe von kettenübertragungsaktiven Stoffen, d.h. von Kettenreglern oder Photoinifertern, bewirkt eine Verringerung der Netzwerkdichte des Polyreaktionsnetzwerkes, bei nahezu unveränderter Reaktivität der Polyreaktionsharzmischung. Kettenregler und Photoiniferter werden vorzugsweise in einer Menge von jeweils 0,005 bis 25 Gew.-% und besonders bevorzugt 0,01 bis 10 Gew.-% bezogen auf die Komponente (a) eingesetzt.

Als Entbinderungsbeschleuniger lassen sich auch Comonomere einsetzen, die zu einer Verringerung der thermischen Stabilität von Polymernetzwerken führen. Hierzu eignen sich Comonomere, die thermisch labile Gruppen enthalten, wie z.B. Peroxid-, Azo- oder Urethangruppen, welche während des stereolithographischen Prozesses in das Polymernetzwerk eingebaut werden und dann im thermischen Entbinderungsprozess den Abbau des Polymernetzwerkes beschleunigen. Ein bevorzugtes Beispiel für ein polymerisationsfähiges Peroxid ist 4,4'-Divinylbenzoylperoxid, das durch Umsetzung von 4-Vinylbenzoylchlorid mit Natriumperoxid zugänglich ist. Ein bevorzugtes Beispiel für eine polymerisationsfähige Azo-Verbindung ist der Ester aus 2-Hydroxyethylmethacrylat und der 4,4'-Azobis-(4-cyano-valeriansäure). Bevorzugte thermisch labile Urethane sind aus Diisocyanaten zugänglich, beispielsweise durch Umsetzung von 2,2,4-Trimethylhexamethylendiisocyanat (TMDI) oder Toluylendiisocyanat (TDI) mit Hydroxypropylacrylat (HPA) oder 2-Hydroxyethylacrylat (HEA). Ein weiteres Beispiel für einen thermisch labilen Monomerbaustein stellt α,α,α',α'-Tetramethyl-1,4-benzen-dime-thylacrylat dar, dessen Einbau in ein Michael-Addition-Netzwerke beispielsweise von Diacrylaten und Diacetoacetaten in Gegenwart katalytischer Säuremengen zu einem beschleunigten Abbau des Polymernetzwerks führt.

Außerdem sind als Entbinderungsbeschleuniger Comonomere geeignet, deren Polyreaktionsprodukte thermisch leicht abbaubar sind. Für radikalische Polymerisationsharze sind Comonomere bevorzugt, die wie α-Methylstyrol eine niedrige ceiling-Temperatur T_{c} aufweisen. Die ceiling-Temperatur ist die Grenztemperatur, bei der die Polymerisation mit der Depolymerisation im Gleichgewicht steht, und lässt sich aus dem Quotienten der Polymerisationsenthalpie und der Polymerisationsentropie berechnen (vgl. H.-G. Elias, Makromoleküle, Bd 1, 6. Aufl., Wiley-VCH, Weinheim etc. 1999, 193 ff.). Beispielsweise beträgt T_{c} für α-Methylstyrol 61°C. Die ceiling-Temperatur T_{c} von Polytetrahydrofuran (PTHF) liegt bei 80°C. Dementsprechend kann z.B. durch Verwendung von Telechelen, insbesondere PTHF-Di(meth)acrylat-Telechelen als Comonomer die Abbaubarkeit von Poly(meth)acrylatnetzwerken beschleunigt werden. Erfindungsgemäß sind Comonomere mit einer ceiling-Temperatur von -10 bis 150°C, vorzugsweise 10 bis 150°C und besonders bevorzugt 20 bis 130°C besonders geeignet. Die Comonomere werden vorzugsweise in einer Menge von 0,1 bis 30 Gew.-% und besonders bevorzugt 0,5 bis 20 Gew.-% bezogen auf die Komponente (a) eingesetzt.

Weiterhin können die erfindungsgemäßen Schlicker Additive enthalten, die beim Entbinderungsprozess den oxidativen Abbau der Polymermatrix fördern, wie z.B. bei Raumtemperatur stabile Peroxide, oder auch katalytisch wirksame Komponenten, die eine katalytische Entbinderung ermöglichen. Neben Peroxiden eignen sich auch andere Substanzen, die oxidierend wirken, wie z.B. Salpetersäure, oder die Oxidationsmittel abspalten oder bilden.

Zusätzlich können die erfindungsgemäß Schlicker **Entschäumungsmittel** und/oder Hautverhinderungsmittel enthalten, die die Schaumbildung bei der Herstellung der Schlicker bzw. die Bildung einer Haut während der Verarbeitung der Schlicker verhindern. Entschäumungs-und/oder Hautverhinderungsmittel werden vorzugsweise in eine Menge von jeweils 0 bis 5 Gew.-%, besonders bevorzugt 0,1 bis 2 Gew.-% in der organischen Matrix eingesetzt, bezogen auf die Masse der Komponente (a).

Die rheologischen Eigenschaften der erfindungsgemäßen Schlicker werden vorzugsweise so eingestellt, dass ihre Viskosität im Bereich von 200 bis 200.000 mPas, besonders bevorzugt 500 bis 100.000 mPas liegt. Die Viskosität wird bei der gewünschten Verarbeitungstemperatur des Schlickers mit einem Platte-Platte-Viskosimeter bei einer Scherrate von 20/s bestimmt. Die Verarbeitungstemperatur liegt vorzugsweise im Bereich von 10 bis 100°C, besonders bevorzugt 15 bis 60°C und ganz besonders bevorzugt zwischen 20 bis 50°C.

Die erfindungsgemäßen Schlicker weisen vorzugsweise folgende Zusammensetzung auf:
- 5 bis 65 Gew.-%, vorzugsweise 9 bis 57 Gew.-%, besonders bevorzugt 10 bis 40 Gew.- % Monomer (a);
- 0,001 bis 1,0 Gew.-%, vorzugsweise 0,01 bis 1,0 Gew.-%, besonders bevorzugt 0,05 bis 1,0 Gew.-% Photoinitiator (b);
- 33 bis 90 Gew.-%, vorzugsweise 40 bis 88 Gew.-%, besonders bevorzugt 56 bis 86 Gew.-% Keramik-, Glas- und/oder Glaskeramikpartikel (c);
- ggf. 1 bis 30 Gew.-%, vorzugsweise 2 bis 15 Gew.-%, besonders bevorzugt 3 bis 10 Gew.-% nichtionisches Tensid (d).

Die Keramik-, Glas- und/oder Glaskeramikpartikel (c) sind vorzugsweise auf die oben beschriebene Weise eingefärbt, also beispielsweise mit färbenden Pigmenten gemischt.

Weiterhin enthalten die Schicker vorzugsweise:
- 0 bis 0,2 Gew.-%, vorzugsweise 0 bis 0,05 Gew.-%, besonders bevorzugt 0 bis 0,02 Gew.-% Farbstoff(e) zur Einfärbung der Schlicker; und/oder
- 0,1 bis 5 Gew.-%, vorzugsweise 0,2 bis 2 Gew.-%, besonders bevorzugt 0,5 bis 1,5 Gew.-% Oberflächenmodifizierungsmittel; und/oder
- 0 bis 50 Gew.-%, vorzugsweise 5 bis 20 Gew.-%, besonders bevorzugt 5 bis 10 Gew.- % Lösungsmittel.

Wenn nicht anders angegeben beziehen sich alle Angaben auf das Gesamtgewicht des Schlickers. Die zur Einfärbung der Keramik-, Glas- und/oder Glaskeramikpartikel verwendeten farbgebenden Komponenten sind in den für die Komponente (c) genannten Mengen enthalten.

Erfindungsgemäß werden zur Herstellung von Corestruktur und Shellstruktur Schlicker der gleichen Kategorie verwendet, also Glas-, Glaskeramik- oder Keramikschlicker, wobei die Schlicker unterschiedlich gefärbt sein können. Gemäß einer besonders bevorzugten Ausführungsform der Erfindung werden zur Herstellung von Core- und Shellstruktur Glaskeramik und ganz besonders bevorzugt Lithiumdisilikat-Glaskeramik-Schlicker eingesetzt, die sich nach dem Sintern nur in Farbe und Opazität unterscheiden und im Übrigen die gleiche Zusammensetzung aufweisen. Auf diese Weise wird eine besonders gut Übereinstimmung zwischen den Wärmeausdehnungskoeffizienten und dem Sinterschrumpf der Schlicker erzielt. Wenn ästhetische Aspekte im Hintergrund stehen, können Core- und Shellstruktur auch aus demselben Schlicker hergestellt werden.

Es kann vorteilhaft sein, zur Herstellung der Shellstruktur einen Schlicker zu verwenden, der einen minimal geringeren oder höheren Gehalt an Keramik-, Glas- und/oder Glaskeramikpartikeln (c) als der Schlicker zur Herstellung der Corestruktur enthält. Vorzugsweise beträgt die Abweichung maximal ± 1 Gew.-%. Dies führt zu einem geringfügig größerem Sinterschrumpf der Shellstruktur. Der hierbei erzeugte Spannung bewirkt eine Stabilisierung der Dentalrestauration. Das ist besonders bei einteiligen Shellstrukturen der Fall, die den Core vollständig begrenzen und eine geschlossene Hülle bilden. Eine geringfügig abweichende Sinterschwindung ist besonders dann vorteilhaft, wenn auf einen Fügeschlicker verzichtet werden soll.

Es kann außerdem vorteilhaft sein, zur Herstellung der Shellstruktur einen Schlicker zu verwenden, der Keramik-, Glas- und/oder Glaskeramikpartikel mit einer geringeren Erweichungstemperatur (Glastemperatur) als der zur Herstellung des Cores verwendete Schlicker enthält. Die Differenz der Erweichungstemperaturen ist vorzugsweise ≤ 50°C. Hierdurch kann der Glanz der Dentalrestauration verbessert werden. Außerdem kann bei unterschiedlichen Erweichungstemperaturen von Core- und Shellmaterial das Sintern so gesteuert werden, dass die Porosität des Cores höher als die der Shellstruktur ist. Generell wird beim Sintern eine möglichst hohe Dichtheit der Shellstruktur angestrebt. Liegt die Dichtheit des Cores unter der der Shellstruktur, erscheint er im Vergleich zur Shellstruktur opaker zu sein, was der Restauration ein natürlicheres Aussehen verleiht.

Zum Zusammenfügen der Grünkörper von Core und Shellstruktur wird vorzugsweise ebenfalls ein Schlicker mit der oben definierten Zusammensetzung verwendet. Farbe und Transparenz dieses Fügeschlickers werden vorzugsweise so gewählt, dass sie zwischen der Farbe und Transluzenz von Core und Shellstruktur liegen. Falls ein Fügeschlicker eingesetzt werden soll, wird vorzugsweise zumindest stellenweise ein Fügespalt zwischen Core und Shellstruktur vorgesehen, vorzugsweise mit einer Breite von 10 bis 300 µm. Das Zusammenfügen der Grünkörper von Core und Shell kann beispielsweise dadurch erfolgen, dass der Fügeschlicker auf den Grünkörper des Cores aufgebracht wird und dann der oder die Grünkörper der Shellstruktur unter leichtem Druck auf den Coregrünkörper aufgesetzt werden. Anschließend werden Überschüsse des Fügeschlickers entfernt.

Die Verwendung von Fügeschlicker und Fügespalt haben den Vorteil, dass sog. Befestigungsstege, Stützstrukturen etc., die beim Druck der Formkörper gebildet werden, vor dem Fügen nur soweit entfernt werden müssen, wie sie die Maße des Fügespalts überschreiten. Kleinere Überreste und Ungenauigkeiten werden durch den Fügespalt und den Fügeschlicker kompensiert.

Die Fügeschlicker dienen primär dazu, die Teile nach dem Fügen zu fixieren. Sie sollen standfest sein, so dass sie während des Auftragens nicht abfließen und eine standfeste Schlickerschicht bilden, und nach dem Fügen über eine ausreichende Festigkeit und Adhäsionswirkung verfügen. Beim Zusammenfügen werden die zu fügenden Teile formschlüssig zusammengeführt. Das Fügen kann bei Bedarf beispielsweise durch einen Rütteltisch erleichtert werden. Die durch die Fügeschlicker bewirkte Fixierung der Teile ist für die weitere Bearbeitung der Grünkörper meist ausreichend, so dass eine Photohärtung der Fügeschlicker nicht zwingend erforderlich ist aber vorteilhaft sein kann.

Die erfindungsgemäßen Schlicker sind im flüssigen Zustand sedimentationsstabil. Sie ergeben eine gute Grünkörperfestigkeit und hohe Formstabilität, Genauigkeit und Präzision nach dem Entbindern, Sintern und Reinigen. Die Grünkörper haben eine hohe Gründichte und lassen sich ohne Verformung, Riss- oder Spannungsbildung entbindern. Sie führen beim Sintern zu hochfesten Keramiken, die für dentale Zwecke geeignet sind. Beim Sintern kann ohne weiteres eine Dichte von > 98% der theoretischen Dichte erzielt werden. Die Schlickern eignen sich besonders zur Herstellung von Keramik- oder Glaskeramikformteilen, insbesondere zur Herstellung von Dentalrestauration, wie z.B. Inlays, Onlays, Veneers, Kronen, Brücken oder Gerüsten.

Die Herstellung der Grünlinge erfolgt vorzugsweise durch Stereolithographie. Der stereolithographische Druck erfolgt computergestützt auf der Basis von CAD-Daten. Die erforderlichen Daten werden auf bekannte Weise durch optische oder mechanische Digitalisierung im Munde des Patienten (intraoral) oder anhand von Modellen (extraoral) gewonnen. Auf Basis dieses Datensatzes werden dann am Computer Core und Shellstruktur der Dentalrestauration konstruiert. Hierbei kann auf in Datenbanken hinterlegte Zahnformen zurückgegriffen werden. Anschließend werden softwaregesteuert zwei Datensätze erstellt, die dazu dienen die Core- und die Shellstruktur unabhängig voneinander zu drucken. Bei der Konstruktion der Restauration wird das materialbedingte Schrumpfungsverhalten entsprechend berücksichtigt.

Die durch den Druckvorgang hergestellten Grünkörper haben bereits eine relativ hohe Festigkeit und können problemlos gehandhabt werden. Die Grünkörper von Core und Shellstruktur werden vorzugsweise gereinigt und dann zu dem Grünkörper der Dentalrestauration zusammengesetzt. Aufgrund der weitgehenden Übereinstimmung des Sinterschrumpfs und der Wärmeausdehnungskoeffizienten können die Grünkörper passgenau hergestellt werden, so dass sie nach dem Zusammenfügen auch ohne Fügeschlicker form- und/oder kraftschlüssig zusammenhalten und entbindert und gesintert werden können. Vorzugsweise wird zum Zusammenfügen der Bauteile aber ein Fügeschlicker eingesetzt, insbesondere dann, wenn die Shellstruktur die Corestruktur nicht vollständig umfasst und/oder mehrteilig ist.

Die stereolithographische Herstellung von Core und Shellstruktur hat den Vorteil, dass die Formkörper mit großer Präzision hergestellt werden können und beispielsweise keine Frässpuren etc. aufweisen. Vorteilhaft ist auch, dass nur ein Entbinderungs- und Sintervorgang erforderlich ist, was den Zeitaufwand zur Herstellung der Dentalrestauration erheblich verringert.

Nach dem Sintern können die Dentalrestaurationen optional mit einer Glasur versehen und mit dentalkeramischen Malfarben individuell charakterisiert werden.

Das erfindungsgemäße Verfahren und die erfindungsgemäß verwendeten Materialien eignen sich zur Herstellung von Dentalrestaurationen zur Versorgen mehrerer Zähne, wie Brücken, und insbesondere zur Herstellung von Dentalrestaurationen zur Versorgung eines Zahnes, wie Inlays, Onlays, Veneers und ganz besonders Kronen. Durch den mehrschichtigen Aufbau der Restaurationen lassen sich verbesserte ästhetischen Eigenschaften erzielen. Durch das Zusammenfügen von Core- und Shellstruktur im Grünzustand und das anschließende gemeinsame Sintern der Komponenten bedingt der mehrschichtige Aufbau aber keinen nennenswerten Mehraufwand im Vergleich zur Herstellung einteiliger Restaurationen. Die erfindungsgemäß bevorzugten Schlicker ergeben zudem eine hohe Grünfestigkeit, die eine sichere Handhabung der Grünkörper ermöglicht und eine hohe Stabilität beim Entbindern gewährleistet. Außerdem lassen sich die Grünkörper auch nach dem Fügen sehr gut entbindern.

Die Erfindung wird im Folgenden anhand von Zeichnungen und Ausführungsbeispielen näher erläutert.
**Figur 1** zeigt eine am Computer konstruierte Corestruktur einer Frontzahnkrone mit Mamelons in Frontansicht.
**Figur 2** zeigt die zu der im Figur 1 gezeigten Corestruktur passende Shellstruktur in Rückansicht.
**Figur** 3 zeigt die gedruckte, entbinderte und separat gesinterte Shell- (links) und Corestruktur (rechts). Shell- und Corestruktur wurden separat gesintert, um Farbunterschiede deutlicher zu zeigen.
**Figur 4** zeigt eine andere Ansicht der in Figur 3 gezeigten Shell- (links) und Corestruktur (rechts).
**Figur 5** zeigt eine Frontzahnkrone, die aus separat gedruckter Core- (Dentin) und Shellstrukture (Schneide) zusammengesetzt ist. Die gedruckten Teile wurden im Grünzustand gefügt und gemeinsam entbindert und gesintert.
**Figur 6** zeigt die in Figur 5 gezeigte Frontzahnkrone nach dem Polieren und Glasieren.

### Ausführungsbeispiele

### Beispiel 1

### Herstellung von Core- und Shellstruktur einer Frontzahnkrone

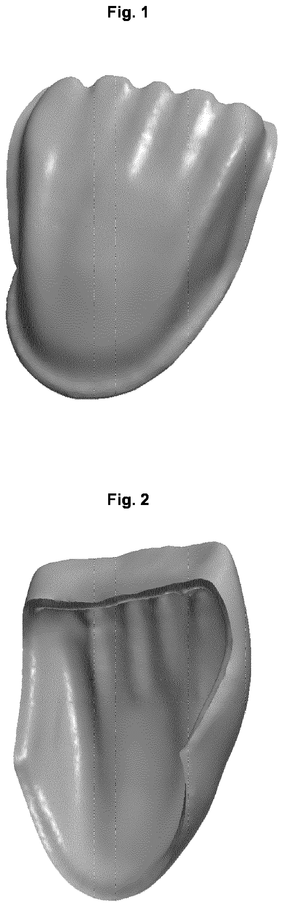

Unter Verwendung einer üblichen CAD-Software (EXOCAD v3.2; exocad GmbH, Rosa-Parks-Str. 2, D-64295 Darmstadt, Deutschland) wurde am Computer eine Krone für einen Schneidezahn konstruiert. Der Datensatz wurde dann in zwei Teildatensätze unterteilt. Der erste Teildatensatz definiert die in Figur 1 gezeigte Corestruktur der Krone und der zweite Teildatensatz die in Figur 2 gezeigte einteilige Shellstruktur. Zwischen Core- und Shellstruktur wurde ein Fügespalt mit einer Breite von 35 µm vorgesehen. Sowohl Core- als auch Shellstruktur weisen Höcker auf, die die natürlichen Mamelons nachahmen. Diese Strukturen erleichtern ein Zusammenfügen von Core und Shellstruktur und verleihen der Dentalrestauration nach dem Fügen eine natürlichen Aussehen.

Die Teildatensätze wurden mit einer üblichen CAM-Software (PrograPrint CAM-Software V1.2, Ivoclar Vivadent AG, Bendererstr. 2, 9494 Schaan, Liechtenstein) getrennt in Bearbeitungsdaten für eine stereolithographische Druckmaschine (Prograprint PR5; Ivoclar Vivadent AG) umgewandelt. Der Drucker wurde in einer Klimakammer bei 40°C betrieben, um die Fließfähigkeit der Schlicker zu gewährleisten. Anschließend wurden die Corestruktur und die Shellstruktur computergesteuert getrennt voneinander gedruckt. Die hierzu verwendeten Schlicker hatten die folgende Zusammensetzung:

| **Komponente** | **ShellSchlicker** | **CoreSchlicker** |
|---|---|---|
| Tricyclodecandioldiacrylat (SR833S) | 9,12 | 9,12 |
| Bisphenol-A-dimethacrylat mit 3 Ethoxygruppen (SR-348c) | 12,125 | 11,61 |
| Photoinitiator (Ivocerin) | 0,23 | 0,23 |
| 4-(4-Nitrophenylazo)anilin 3 (Disperse orange 3, CAS 730-40-5) | 0,015 | 0,015 |
| Rheologieadditiv (Solplus D540, CAS 1000871-74-8, Firma Lubrizol) | 4,17 | 4,17 |
| Benzylbenzoat (Lösungsmittel) | 4,5 | 4,5 |
| Diethylenglycoldibenzoat (Lösungsmittel) | 0,34 | 0,34 |
| Lithiumdisilikat-Glaspulver¹⁾ (e.max Press, Farbe Schneide S2, D50=6 µm²⁾) | 69,5 | 0 |
| Lithiumdisilikat-Glaspulver¹⁾ (e.max Press, Farbe Dentin MT A3, D50=6 µm²) | 0 | 70,015 |
| Summe | 100 | 100 |

| | | |
|---|---|---|
| ¹⁾ gemäß EP 0 827 941 A1 ²⁾ Partikelgrößenbereich: 500 nm bis 50 µm | | |

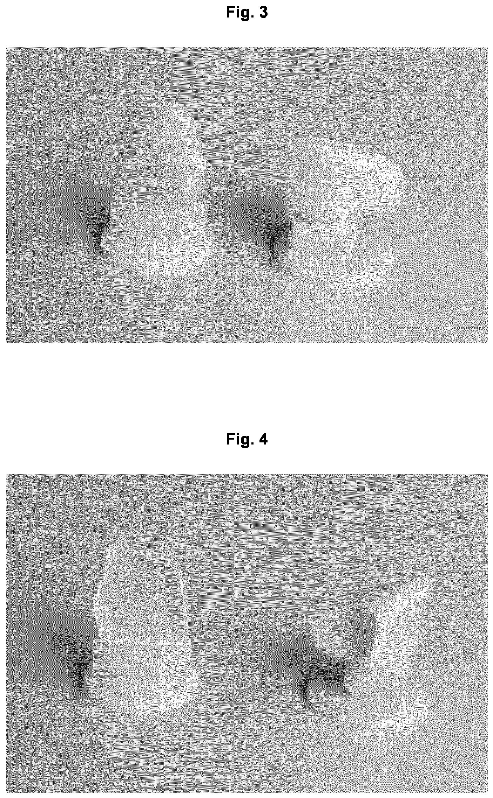

Die gedruckten Grünkörper wurden durch Abspülen mit Wasser gereinigt und mit Pressluft trockengeblasen. Die erhaltenen Grünkörper wurden dann durch Erhitzen mit einer Heizrate von 0.2 K/min auf 500°C für 30 Minuten in einem Ofen (Nabertherm L9/11 BO; Firma Lilienthal Bremen, Deutschland) entbindert und anschließend durch Erhitzen mit einer Heizrate von 10 K/min auf 890°C für 5 Minuten in einem Sinterofen (Programat P510; Firma Ivoclar Vivadent AG; Schaan, Liechtenstein) gesintert. Die gesinterten Strukturen sind den Figuren 3 und 4 gezeigt.

### Beispiel 2

### Herstellung einer Frontzahnkrone

Auf die in Beispiel 1 beschriebene Weise wurden eine Krone für einen Frontzahn konstruiert und mit den in der folgenden Tabelle beschriebenen Schlickern hergestellt.

| **Komponente** | **ShellSchlicker** | **CoreSchlicker** | **Fügeschlicker** |
|---|---|---|---|
| Tricyclodecandioldiacrylat (SR833S) | 9,12 | 9,12 | 9,12 |
| UDMA | 12,125 | 11,61 | 11,875 |
| Photoinitiator (Ivocerin) | 0,23 | 0,23 | 0,23 |
| 4-(4-Nitrophenylazo)anilin 3 (Disperse orange 3, CAS 730-40-5) | 0,015 | 0,015 | 0,015 |
| Rheologieadditiv (Solplus D540, CAS 1000871-74-8, Firma Lubrizol) | 4,17 | 4,17 | 4,17 |
| Benzylbenzoat (Lösungsmittel) | 4,5 | 4,5 | 4,5 |
| Diethylenglycoldibenzoat (Lösungsmittel) | 0,34 | 0,34 | 0,34 |
| Lithiumdisilikat-Glaspulver¹⁾ (e.max Press, Farbe Schneide S2, D50=6 µm²⁾) | 69,5 | 0 | 0 |
| Lithiumdisilikat-Glaspulver¹⁾ (e.max Press, Farbe Dentin MT A3, D50=6 µm²⁾) | 0 | 70,015 | 0 |
| Lithiumdisilikat Glaspulver¹⁾ (e.max Press, Farbe Schneide S2, D50=6 µm²⁾) | 0 | 0 | 69,75 |
| Summe | 100 | 100 | 100 |

| | | | |
|---|---|---|---|
| ¹⁾ gemäß EP 0 827 941 A1 ²⁾ Partikelgrößenbereich: 500 nm bis 50 µm | | | |

Die gedruckten Grünkörper wurden durch Abspülen mit Wasser gereinigt und mit Pressluft trockengeblasen. Bei der Schneide wurde die Stützstruktur mit einer rotierenden Trennscheibe (zahntechnisches Standardwerkzeug) entfernt. Danach wurde der Fügeschlicker auf die Klebeflächen von Core und Shell aufgetragen, beide Formen formschlüssig zusammengedrückt und der überschüssige Fügeschlicker dann entfernt.

Die gefügten Grünkörper wurden anschließend einer Lichthärtung mit einem dentalen Lichthärtegerät (PrograPrint Cure, Programm Wax, Firma Ivoclar Vivadent AG) unterworfen, um den Polymerisationsgrad zu erhöhen. Hierbei wurde auch der Fügeschlicker gehärtet.

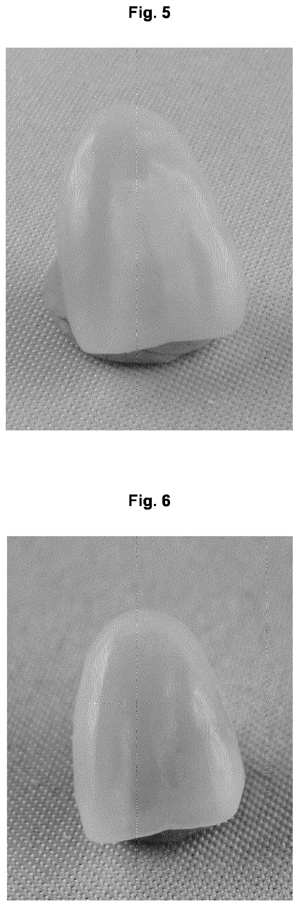

Die erhaltenen Grünkörper wurden dann durch Erhitzen mit einer Heizrate von 0,2 K/min auf 500°C für 30 Minuten in einem Ofen (Nabertherm L9/11 BO, Firma Lilienthal Bremen, Deutschland) entbindert und anschließend durch Erhitzen mit einer Heizrate von 10 K/min auf 890°C für 5 Minuten in einem Sinterofen (Programat P510; Firma Ivoclar Vivadent AG; Schaan, Liechtenstein) gesintert. Die erhaltene Krone ist in Figur 5 gezeigt. Die in Figur 5 gezeigte Krone wurde anschließend poliert. Die fertige Krone ist in Figur 6 dargestellt.

Der zur Herstellung der Shellstruktur verwendete Schlicker war etwas weniger gefüllt als der zur Herstellung des Cores verwendete Schlicker. Er wies daher einen geringfügig größeren Sinterschrumpf als der Coreschlicker auf, was beim Sintern zu einer Verbesserung des Verbunds von Core- und Shellstruktur führte. Der Füllgrad des Fügeschlickers lag zwischen dem Füllgrad von Core- und Shellschlicker, wodurch ein guter Übergang zwischen Core und Shell erzielt wurde.

## Patentansprüche

1. Verfahren zur Herstellung einer vollkeramischen Dentalrestauration mit einer einteiligen Corestruktur und einer ein- oder mehrteiligen Shellstruktur, bei dem man
in einem ersten Schritt ein digitales Konstruktionsmodell der Dentalrestauration konstruiert, man anschließend den hierbei erhaltenen CAD-Datensatz der Restauration in mindestens zwei separate CAD-Teildatensätze unterteilt, wobei ein erster Teildatensatz die Konturen der Corestruktur und ein zweiter Teildatensatz die Konturen der Shellstruktur definiert und man
dann die Dentalrestauration mittels der erstellten Datensätze fertigt, **dadurch gekennzeichnet, dass**
man in einem folgenden Schritt mittels des ersten CAD-Teildatensatzes einen Grünling der Corestruktur der Restauration herstellt, indem man einen ersten Schlicker durch lokales Einbringen von Strahlungsenergie unter Ausbildung der geometrischen Form der Corestruktur aushärtet,
man in einem weiteren Schritt mittels des zweiten CAD-Teildatensatzes einen Grünling der Shellstruktur der Restauration herstellt, indem man einen zweiten Schlicker durch lokales Einbringen von Strahlungsenergie unter Ausbildung der geometrischen Form der Shellstruktur aushärtet,
man in einem folgenden Schritt die Core- und die Shellstruktur im Grünzustand zusammenfügt, um einen Grünling der Dentalrestauration zu erhalten,
man in einem weiteren Schritt den Grünling der Dentalrestauration einer Wärmebehandlung zur Entfernung des Bindemittels (Entbinderung) unterwirft, um einen Braunling der Dentalrestauration zu erhalten, und
man dann den Braunling der Dentalrestauration sintert, um die fertige Dentalrestauration zu erhalten.

2. Verfahren nach Anspruch 1, bei dem man den CAD-Teildatensatz der Shellstruktur in zwei oder mehr CAD-Shellstrukturteildatensätze unterteilt, die jeweils einen Teil der Kontur der Shellstruktur umfassen, man einen zweiten Schlicker mittels des ersten CAD-Shellstrukturteildatensatzes durch lokales Einbringen von Strahlungsenergie unter Ausbildung der geometrischen Form einer ersten Komponente der Shellstruktur aushärtet, man getrennt davon einen weiteren Schlicker mittels des zweiten CAD-Shellstrukturteildatensatzes durch lokales Einbringen von Strahlungsenergie unter Ausbildung der geometrischen Form einer zweiten Komponente der Shellstruktur aushärtet, und man diesen Schritt der Anzahl der CAD-Shellstrukturteildatensätze entsprechend wiederholt und pro Wiederholung einen Grünling einer weiteren Komponente der Shellstruktur herstellt, und man anschließend die Corestruktur und die Komponenten der Shellstruktur im Grünzustand zusammenfügt, um einen Grünling der Dentalrestauration zu erhalten.

3. Verfahren nach Anspruch 2, bei dem man zur Herstellung der Komponenten der Shellstruktur denselben oder vorzugsweise für jede Komponente einen anderen Schlicker, besonders bevorzugt einen unterschiedlich gefärbten, Schlicker verwendet.

4. Verfahren nach einem der Ansprüche 1 bis 3, wobei man zur Herstellung der Corestruktur und zur Herstellung der Shellstruktur Schlicker verwendet, die sich in Farbe und Opazität unterscheiden und im Übrigen die gleiche Zusammensetzung aufweisen.

5. Verfahren nach einem der Ansprüche 1 bis 4, wobei man zur Herstellung der Shellstruktur einen Schlicker verwendet, der einen minimal geringeren oder minimal höheren Gehalt an Keramik-, Glas- und/oder Glaskeramikpartikeln (c) als der Schlicker zur Herstellung de Corestruktur enthält.

6. Verfahren nach einem der Ansprüche 1 bis 5, bei dem man die Grünlinge der Core- und der Shellstruktur unter Verwendung eines Fügeschlickers zusammenfügt und man den Fügeschlicker nach dem Fügen durch Polymerisation härtet.

7. Verfahren nach einem der Ansprüche 1 bis 6, bei dem man den Grünling der Dentalrestauration entbindert, indem man das Bindemittel durch Erhitzen des Grünlings auf eine Temperatur von 90°C bis 600°C entfernt.

8. Verfahren nach einem der Ansprüche 1 bis 7, bei dem man den Braunling bei einer Temperatur 650 bis 1800°C sintert.

9. Verfahren nach einem der Ansprüche 1 bis 8, bei dem man zur Herstellung der Grünlinge jeweils einen Schlicker verwendet, der
(a) 5 bis 65 Gew.-% mindestens eines radikalisch polymerisierbaren Monomers,
(b) 0,001 bis 1,0 Gew.-% mindestens eines Photoinitiators und
(c) 33 bis 90 Gew.-% Keramikpartikel und/oder Glaspartikel und/oder Glaskeramik-partikel enthält,
jeweils bezogen auf die Gesamtmasse des Schlickers.

10. Verfahren nach Anspruch 9, bei dem man zur Herstellung der Grünling jeweils einen Schlicker verwendet, der Lithiumdisilikat-Glaskeramik-Partikel und/oder Glaspartikel für eine Lithiumdisilikat-Glaskeramik enthält.

11. Verfahren nach Anspruch 10, bei dem man zur Herstellung der Grünlinge jeweils einen Schlicker verwendet der ein Glas- oder Glaskeramikpulver mit der folgenden Zusammensetzung enthält:
| | |
|---|---|
| SiO₂ | 57,0 bis 80,0 Gew.-% |
| Al₂O₃ | 0 bis 5,0 Gew.-% |
| La₂O₃ | 0,1 bis 6,0 Gew.-% |
| MgO | 0 bis 5,0 Gew.-%, insbesondere 0,1 bis 5,0 Gew.-% |
| ZnO | 0 bis 8,0 Gew.-% |
| K₂O | 0 bis 13,5 Gew.-% |
| Li₂O | 11,0 bis 19,0 Gew.-% |
| P₂O₅ | 0 bis 11,0 Gew.-% |
| Farbkomponenten | 0 bis 8,0 Gew.-% |
| Zusatzkomponenten | 0 bis 6,0 Gew.-% |
wobei
| | |
|---|---|
| Al₂O₃ + La₂O₃ | 0,1 bis 7,0 Gew.-% und |
| MgO + ZnO | 0,1 bis 9,0 Gew.-% |
ausmachen und wobei die Farbkomponenten aus glasfärbenden Oxiden und/oder Farbkörpern in den folgenden Mengen gebildet sind:
| | |
|---|---|
| glasfärbende Oxide | 0 bis 5,0 Gew.-% und |
| Farbkörper | 0 bis 5,0 Gew.-%. |

12. Verfahren nach Anspruch 11, bei dem das oder die glasfärbenden Oxide aus TiO₂, CeO₂ und/oder Fe₂O₃ und/oder der oder die Farbkörper aus dotierten Spinellen und/oder dotiertem ZrO₂ und/oder die Zusatzkomponente(n) aus B₂O₃, F, Na₂O, ZrO₂, BaO und/oder SrO ausgewählt sind.

13. Verfahren nach einem der Ansprüche 1 bis 12, bei dem man einen Schlicker verwendet der eine Mischung von Glas- und Glaskeramikpulver enthält.

14. Verfahren nach einem der Ansprüche 9 bis 13, der bei dem man einen Schlicker verwendet, der als radikalisch polymerisierbares Monomer (a) ein aliphatisches Urethandiacrylat, Phthalsäure-HEA-Ester, Pyromellitsäuredi-HEA-Ester, Bisphenol-A-di(meth)acrylat, Bis-G(M)A (ein Additionsprodukt aus (Meth)acrylsäure und Bisphenol-A-diglycidylether), ein ethoxy- oder propoxyliertes Bisphenol-A-di(meth)acrylat, UD(M)A, Triethylenglycoldi(meth)acrylat (TEGD(M)A), Tricyclodecandimetha-noldi(meth)acrylat, ethoxyliertes oder propoxyliertes Trimethylolpropantri(meth)acrylat, Tripropylenglycoldiacrylat oder eine Mischungen davon enthält.

15. Verfahren nach einem der Ansprüche 9 bis 14, der bei dem man einen Schlicker verwendet, der als Photoinitiator (b) Campherchinon (CAS Nr. 10373-78-1) in Kombination mit Ethyl-4-(dimethylamino)benzoat (CAS Nr. 10287-53-3), 2,4,6-Trimethylbenzoyldiphenylphosphinoxid (CAS Nr. 75980-60-8), Ethyl(2,4,6-trimethylbenzoyl)phenyl-phosphinat (CAS Nr. 84434-11-7), Phenylbis(2,4,6-trimethylbenzoyl)phosphinoxid (CAS Nr. 162881-26-7), Bis(2,6-difluoro-3-(1-hydropyrrol-1-yl)phenyl)titanocen (CAS Nr. 125051-32-3), 2-Benzyl-2-(dimethylamino)-4'-morpholinobutyrophenon (CAS Nr. 119313-12-1), 1-Butanon-2-(dimethylamino)-2-(4-methylphenyl)methyl-1-4-(4-morpholinyl)phenyl (CAS-Nr. 119344-86-4), Bis(4-methoxybenzoyl)diethylgermanium oder eine Mischung davon enthält.

## Claims

1. A process for the production of an all-ceramic dental restoration with a one-piece core structure and a one- or multi-part shell structure, comprising
in a first step, constructing a digital model of the dental restoration, dividing the resulting CAD data set into at least two separate CAD partial data sets wherein a first partial data set defines a contour of the core structure and a second partial data set defines a contour of the shell structure,
and fabricating the dental restoration using the data sets, **characterized by**
in a subsequent step, fabricating a green body of the core structure of the restoration using the first partial data set comprising curing a first slurry by locally applying radiation energy to form a geometric shape of the core structure,
in a further step, fabricating a green body of the shell structure of the restoration using the second partial data set comprising curing a second slurry by locally applying radiation energy to form a geometric shape of the shell structure,
in a further step, combining the core and shell structures in the green state in order to obtain a green body of the dental restoration,
in a further step, subjecting the green body of the dental restoration to a heat treatment to remove a binder (debinding) to obtain a brown body of the dental restoration, and
sintering the brown body of the dental restoration to obtain the finished dental restoration.

2. The process according to claim 1, in which the CAD partial data set of the shell structure is divided into two or more CAD shell structure partial data sets, each comprising a part of the contour of the shell structure, a second slurry is cured using a first partial data set of the shell structure by local introduction of radiation energy to form a geometric shape of a first component of the shell structure, separately, a further slurry is cured using a second partial data set of the shell structure by local application of radiation energy to form a geometric shape of a second component of the shell structure, and this step is repeated according to the number of CAD shell structure partial data sets, and with each repetition, a green body of a further component of the shell structure is fabricated, and the core structure and the components of the shell structure are then joined together in the green state to obtain a green body of the dental restoration.

3. The process according to claim 2, in which the same slurry is used to produce the components of the shell structure, or preferably a different slurry is used to produce each component of the shell structure, more preferably a differently colored slurry.

4. The process according to any one of claims 1 to 3, wherein slurries which differ in color and opacity but otherwise have the same composition are used to produce the core structure and to produce the shell structure.

5. The process according to any one of claims 1 to 4, wherein the slurry used to produce the shell structure comprises a minimally lower or minimally higher content of ceramic, glass and/or glass-ceramic particles (c) than the slurry used to produce the core structure.

6. The process according to any one of claims 1 to 5, in which the green bodies of the core structure and the shell structure are joined together using a joining slurry and the joining slurry is cured by polymerization after joining.

7. The process according to any one of claims 1 to 6, wherein the green body of the dental restoration is debinded by removing the binder by heating the green body to a temperature of 90°C to 600°C.

8. The process according to any one of claims 1 to 7, wherein the brown body is sintered at a temperature of 650 to 1800°C.

9. The process according to any one of claims 1 to 8, in which the slurry used to produce each of the green bodies comprises
(a) 5 to 65% by weight of at least one free-radically polymerizable monomer,
(b) 0.001 to 1.0% by weight of at least one photoinitiator and
(c) 33 to 90% by weight of ceramic particles and/or glass particles and/or glass-ceramic particles,
in each case relative to the total mass of the slurry.

10. The process according to claim 9, in which the slurry used to produce each of the green bodies comprises lithium disilicate glass-ceramic particles and/or glass particles for a lithium disilicate glass-ceramic.

11. The process according to claim 10, in which the slurry used to produce each of the green bodies comprises a glass or glass-ceramic powder having the following composition:
| | |
|---|---|
| SiO₂ | 57.0 to 80.0 % by weight |
| Al₂O₃ | 0 to 5.0 % by weight |
| La₂O₃ | 0.1 to 6.0 % by weight |
| MgO | 0 to 5.0 % by weight, in particular 0.1 to 5.0 % by weight |
| ZnO | 0 to 8.0 % by weight |
| K₂O | 0 to 13.5 % by weight |
| Li₂O | 11.0 to 19.0 % by weight |
| P₂O₅ | 0 to 11.0 % by weight |
| Color components | 0 to 8.0 % by weight |
| Additional components | 0 to 6.0 % by weight |
whereby
| | |
|---|---|
| Al₂O₃ + La₂O₃ is | 0.1 to 7.0 % by weight and |
| MgO + ZnO is | 0.1 to 9.0 % by weight |
and wherein the color components are formed from glass coloring oxides and/or color bodies in the following amounts:
| | |
|---|---|
| glass-coloring oxides | 0 to 5.0 % by weight and |
| color bodies | 0 to 5.0 % by weight. |

12. The process according to claim 11, wherein the glass coloring oxide(s) is/are selected from TiO₂, CeO₂ and/or Fe₂O₃ and/or the color body/color bodies is/are selected from doped spinels and/or doped ZrO₂ and/or the additional component(s) is/are selected from B₂O₃, F, Na₂O, ZrO₂ , BaO and/or SrO.

13. The process according to any one of claims 1 to 12, wherein a slurry is used which comprises a mixture of glass and glass-ceramic powder.

14. The process according to any one of claims claim 9 to 13, wherein a slurry is used which comprises, as the free-radically polymerizable monomer (a), an aliphatic urethane diacrylate, phthalic acid HEA ester, pyromellitic acid diHEA ester, bisphenol A di(meth)acrylate, bis-G(M)A (an addition product of (meth)acrylic acid and bisphenol A diglycidyl ether), an ethoxylated or propoxylated bisphenol A di(meth)acrylate, UD(M)A, triethylene glycol di(meth)acrylate (TEGD(M)A), tricyclodecane dimethanol di(meth)acrylate, ethoxylated or propopoxylated trimethylolpropane tri(meth) acrylate, tripropylene glycol diacrylate or a mixture thereof.

15. The process according to any one of claims 9 to 14, wherein a slurry is used which comprises, as the photoinitiator (b), camphorquinone (CAS No. 10373-78-1) in combination with ethyl 4-(dimethylamino)benzoate (CAS No. 10287-53-3), 2,4,6-trimethylbenzoyldiphenylphosphine oxide (CAS No. 75980-60-8), ethyl (2,4,6-trimethylbenzoyl)phenylphosphinate (CAS No. 84434-11-7), phenylbis(2,4,6-trimethylbenzoyl)phosphine oxide (CAS No. 162881-26-7), bis(2,6-difluoro-3-(1-hydropyrrol-1-yl)phenyl)titanocene (CAS No. 125051-32-3), 2-benzyl-2-(dimethylamino)-4'-morpholinobutyrophenone (CAS No. 119313-12-1), 1-butanone-2-(dimethylamino)-2-(4-methylphenyl)methyl-1-4-(4-morpholinyl)phenyl (CAS No. 119344-86-4), bis(4-methoxybenzoyl)diethylgermanium or a mixture thereof.

## Revendications

1. Procédé de fabrication d'une restauration dentaire entièrement céramique avec une structure centrale monobloc et une structure de coque monobloc ou multibloc, selon lequel
dans une première étape on construit un modèle de construction numérique de la restauration dentaire, on divise ensuite l'ensemble de données CAO de la restauration obtenu ici en au moins deux ensembles de données CAO partiels distincts, dans lequel un premier ensemble de données partiel définit les contours de la structure centrale et un second ensemble de données partiel définit les contours de la structure de coque et on
produit ensuite la restauration dentaire au moyen des ensembles de données créés, **caractérisé en ce que**
dans une étape suivante on réalise au moyen du premier ensemble de données CAO partiel un corps crû de la structure centrale de la restauration en durcissant une première barbotine par apport local d'énergie de rayonnement ce qui entraîne la réalisation de la forme géométrique de la structure centrale,
dans une étape supplémentaire on fabrique au moyen du second ensemble de données CAO partiel un corps crû de la structure de coque de la restauration en durcissant une seconde barbotine par apport local d'énergie de rayonnement ce qui entraîne la réalisation de la forme géométrique de la structure de coque,
dans une étape suivante on assemble la structure centrale et la structure de coque à l'état de corps crû pour obtenir un corps crû de la restauration dentaire,
dans une étape supplémentaire, on soumet le corps crû de la restauration dentaire à un traitement thermique pour éliminer le liant (déliantage), afin d'obtenir une pièce brune de la restauration dentaire, et
on fritte ensuite la pièce brune de la restauration dentaire pour obtenir la restauration dentaire finie.

2. Procédé selon la revendication 1, selon lequel on divise l'ensemble de données CAO partiel de la structure de coque en deux ensembles de données partiels de structure de coque CAO ou plus, qui comprennent chacun une partie du contour de la structure de coque, on durcit une seconde barbotine au moyen du premier ensemble de données partiel de structure de coque CAO par apport local d'énergie de rayonnement ce qui entraîne la réalisation de la forme géométrique d'un premier composant de la structure de coque, on durcit séparément de celui-ci une autre barbotine au moyen du second ensemble de données partiel de structure de coque CAO par apport local d'énergie de rayonnement ce qui entraîne la réalisation de la forme géométrique d'un second composant de la structure de coque, et on répète cette étape en fonction du nombre d'ensembles de données partiels de structure de coque CAO et on fabrique à chaque répétition un corps crû d'un autre composant de la structure de coque, et on assemble ensuite la structure centrale et les composants de la structure de coque à l'état de corps crû pour obtenir un corps crû de la restauration dentaire.

3. Procédé selon la revendication 2, selon lequel pour la fabrication des composants de la structure de coque on utilise la même ou de préférence pour chaque composant une barbotine différente, particulièrement préférentiellement une barbotine de couleur différente.

4. Procédé selon l'une quelconque des revendications 1 à 3, dans lequel pour la fabrication de la structure centrale et pour la fabrication de la structure de coque on utilise des barbotines qui se distinguent par leur couleur et leur opacité et qui présentent par ailleurs la même composition.

5. Procédé selon l'une quelconque des revendications 1 à 4, dans lequel pour la fabrication de la structure de coque on utilise une barbotine qui contient une teneur en particules de céramique, de verre et/ou de vitrocéramique (c) très légèrement inférieure ou très légèrement supérieure à celle de la barbotine pour la fabrication de la structure centrale.

6. Procédé selon l'une quelconque des revendications 1 à 5, selon lequel on assemble les corps crûs de la structure centrale et de la structure de coque à l'aide d'une barbotine d'assemblage et on durcit la barbotine d'assemblage après l'assemblage par polymérisation.

7. Procédé selon l'une quelconque des revendications 1 à 6, selon lequel on effectue le déliantage du corps crû de la restauration dentaire en éliminant le liant par chauffage du corps crû à une température de 90 °C à 600 °C.

8. Procédé selon l'une quelconque des revendications 1 à 7, dans lequel on fritte la pièce brune à une température de 650 à 1800 °C.

9. Procédé selon l'une quelconque des revendications 1 à 8, selon lequel pour la fabrication des corps crûs on utilise respectivement une barbotine qui contient
(a) 5 à 65 % en poids d'au moins un monomère radicalement polymérisable,
(b) 0,001 à 1,0 % en poids d'au moins un photo-initiateur et
(c) 33 à 90 % en poids de particules de céramique et/ou de particules de verre et/ou de particules de vitrocéramique, respectivement par rapport à la masse totale de la barbotine.

10. Procédé selon la revendication 9, selon lequel pour la fabrication des corps crûs on utilise respectivement une barbotine qui contient des particules de vitrocéramique au disilicate de lithium et/ou des particules de verre pour une vitrocéramique au disilicate de lithium.

11. Procédé selon la revendication 10, selon lequel pour la fabrication des corps crûs on utilise respectivement une barbotine qui contient une poudre de verre ou de vitrocéramique ayant la composition suivante :
| | |
|---|---|
| SiO₂ | 57,0 à 80,0 % en poids |
| Al₂O₃ | 0 à 5,0 % en poids |
| La₂O₃ | 0,1 à 6,0 % en poids |
| MgO | 0 à 5,0 % en poids, en particulier 0,1 à 5,0 % en poids |
| ZnO | 0 à 8,0 % en poids |
| K₂O | 0 à 13,5 % en poids |
| Li₂O | 11,0 à 19,0 % en poids |
| P₂O₅ | 0 à 11,0 % en poids |
| composants de couleur | 0 à 8,0 % en poids |
| composants additionnels | 0 à 6,0 % en poids |
dans lequel
| | |
|---|---|
| Al₂O₃ + La₂O₃ | representent 0,1 à 7,0 % en poids et |
| MgO + ZnO | 0,1 à 9,0 % en poids |
et dans lequel les composants de couleur sont formés d'oxydes de coloration du verre et/ou de corps de couleur dans les quantités suivantes :
oxydes de coloration0 à 5,0 % en poids et
du verre
corps de couleur 0 à 5,0 % en poids.

12. Procédé selon la revendication 11, selon lequel le ou les oxydes de coloration du verre sont choisis parmi TiO₂, CeO₂ et/ou Fe₂O₃ et/ou le ou les corps de couleur sont choisis parmi les spinelles dopées et/ou le ZrO₂ dopé et/ou le ou les composants supplémentaires sont choisis parmi B₂O₃, F, Na₂O, ZrO₂, BaO et/ou SrO.

13. Procédé selon l'une quelconque des revendications 1 à 12, selon lequel on utilise une barbotine qui contient un mélange de poudre de verre et de vitrocéramique.

14. Procédé selon l'une quelconque des revendications 9 à 13, qui selon lequel on utilise une barbotine qui contient comme monomère radicalement polymérisable (a) un diacrylate d'uréthane aliphatique, ester d'acide phtalique HEA, ester de di-HEA d'acide pyromellitique, bisphénol-A-di (méth)acrylate, bis-G(M)A (un produit d'addition d'acide (méth)acrylique et de bisphénol-A-diglycidyléther), un bisphénol-A-di(méth)acrylate éthoxylé ou propoxylé, UD(M)A, triéthylèneglycoldi(méth)acrylate (TEGD(M)A), tricyclodécandiméthanoldi(méth)acrylate, triméthylolpropantri(méth)acrylate éthoxylé ou propoxylé, tripropylèneglycoldiacrylate ou un mélange de ceux-ci.

15. Procédé selon l'une quelconque des revendications 9 à 14 qui selon lequel on utilise une barbotine qui contient comme photo-initiateur (b) la campherquinone (numéro CAS 10373-78-1) en combinaison avec le 4-(diméthylamino)benzoate d'éthyle (numéro CAS 10287-53-3), le 2,4,6-triméthylbenzoyldiphénylphosphinoxyde (numéro CAS 75980-60-8), le 2,4,6-triméthylbenzoyl)phénylphosphinate d'éthyle (numéro CAS 84434-11-7), le phénylbis(2,4,6-triméthylbenzoyl)phosphinoxyde (numéro CAS 162881-26-7), le bis(2,6-difluoro-3-(1-hydropyrrol-1-yl)phényl)titanocène (numéro CAS 125051-32-3), le 2-benzyl-2-(diméthylamino)-4'-morpholinobutyrophénone (numéro CAS 119313-12-1), le 1-butanone-2-(diméthylamino)-2-(4-méthylphényl)méthyl-1-4-(4-morpholinyl)phényle (numéro CAS 119344-86-4), le bis(4-méthoxybenzoyl)diéthylgermanium ou un mélange de ceux-ci.
